(19) Europäisches Patentamt
European Patent Office
Office européen des brevets

(11) **EP 1 144 452 B1**

(12) **EUROPEAN PATENT SPECIFICATION**

(45) Date of publication and mention
of the grant of the patent:
**11.01.2006 Bulletin 2006/02**

(21) Application number: **99962682.3**

(22) Date of filing: **02.11.1999**

(51) Int Cl.:
*A61K 47/48* (2006.01)

(86) International application number:
**PCT/US1999/025790**

(87) International publication number:
**WO 2000/026256 (11.05.2000 Gazette 2000/19)**

(54) **MODIFIED ANTIBODIES AND ANTIBODY FRAGMENTS WITH INCREASED DURATION OF ACTIVITY**

MODIFIZIERTE ANTIKÖRPER UND ANTIKÖRPER-FRAGMENTE MIT VERLÄNGERTER AKTIVITÄTSDAUER

ANTICORPS ET FRAGMENTS D'ANTICORPS MODIFIES AVEC UNE DUREE D'ACTIVITE ACCRUE

(84) Designated Contracting States:
**AT BE CH CY DE DK ES FI FR GB GR IE IT LI LU MC NL PT SE**
Designated Extension States:
**AL LT LV MK RO SI**

(30) Priority: **03.11.1998 US 185151**

(43) Date of publication of application:
**17.10.2001 Bulletin 2001/42**

(73) Proprietor: **CENTOCOR, INC.**
**Malvern, PA 19355-1307 (US)**

(72) Inventors:
• **HEAVNER, George, A.**
**Malvern, PA 19355 (US)**
• **WEBER, Robert, W.**
**Downingtown, PA 19335 (US)**

(74) Representative: **Lock, Graham James et al**
**Fry Heath & Spence LLP**
**The Gables**
**Massetts Road**
**Horley,**
**Surrey RH6 7DQ (GB)**

(56) References cited:
WO-A-90/06133          WO-A-90/11783
WO-A-96/40250          WO-A-98/25971
WO-A-98/32466          WO-A-98/36778
WO-A-98/48837

• **E MAINOLFI ET AL 'REDUCTION OF IMMUNOGENICITY OF A MURINE ANTI-ICAM-1 ANTIBODY THROUGH PEGYLATION CHEMISTRY ': "In: THE 9TH INTERNATIONAL CONGRESS OF IMMUNOLOGY (abstract book)," , INTERNATIONAL CONGRESS OF IMMUNOLOGY ABSTRACTS,HU,BUDAPEST, PAGE(S) 885 XP002084720 the whole document**
• **TAM S H ET AL: "Abciximab ( ReoPro, chimeric 7E3 Fab) demonstrates equivalent affinity and functional blockade of glycoprotein IIb/IIIa and alpha(v)beta3 integrins." CIRCULATION, (1998 SEP 15) 98 (11) 1085-91. , XP000882678**

Remarks:
•WIPO A3 publication data is not currently available.
•The file contains technical information submitted after the application was filed and not included in this specification

**Description**

BACKGROUND OF THE INVENTION

Platelets play a key role in regulating hemostasis by binding to sites of injury and, upon activation, initiating the biochemical events which result in formation of a clot. However, under certain circumstances platelets can exacerbate pathophysiological conditions such as cardiovascular disease and tumor metastasis. For example, under normal circumstances platelets can adhere to a site of blood vessel injury through the interaction of receptors expressed on the plasma membrane of the platelet with the subendothelial layer, thereby activating the platelets. Platelets can be activated by numerous other stimuli including thrombin, adenosine diphosphate (ADP), contact with artificial surfaces and some immune complexes. Once activated, platelets expose the fibrinogen binding sites on the plasma membrane glycoprotein GPIIb/IIIa and platelet aggregation takes place via fibrinogen bridging. Aggregation of platelets can trigger degranulation of cytoplasmic vesicles, releasing proteins and chemical mediators into the local extracellular space, which can lead to further platelet adhesion, activation and aggregation to generate a clot which prevents the escape of blood cells from the vessel. However, under certain conditions platelet aggregation can occlude a blood vessel leading to ischemia and cellular necrosis. For example, platelet aggregation and clot formation at a ruptured atherosclerotic plaque in a coronary artery can lead to angina or acute myocardial infarction. Further, platelet aggregation and clot formation in blood vessels at sites of endothelial cell injury or denudation, as can be found following angioplasty, can lead to abrupt closure of the vessel necessitating emergency intervention, such as by-pass surgery.

The hemostatic and pathophysiological functions of platelets, which require adhesion and aggregation of the cells, are mediated by the interaction of platelet plasma membrane receptors with ligands. These receptors (e.g., GPIIb/IIIa) are members of the integrin family of receptors which generally bind to matrix proteins (e.g., collagen, fibronectin, fibrinogen, laminin, vitronectin and von Willebrand factor) and are involved in immune function and tumor metastasis in addition to platelet function. Thus, antagonists of certain integrins (e.g., GPIIb/IIIa) hold great promise as therapeutic agents.

Several antagonists of GPIIb/IIIa have been developed including snake venom proteins, small peptides, peptidomimetics, non-peptide compounds and antibodies (Cook, N.S., *et al.*, *Drugs of Future*, 19: 135-159 (1994); Cox, D., *et al.*, *Medicinal Research Reviews*, 14: 195-228 (1994); Coller, B.S., *et al., Thrombosis and Haemostasis,* 74(1) 302-308 (1995)). A fragment of a humanized chimeric antibody, c7E3 Fab (also known as "abciximab" or ReoPro®), that binds GPIIb/IIIa (expressed specifically on platelets), $\alpha_V\beta_3$ (expressed on platelets, endothelial cells and perhaps smooth muscle cells (Felding-Habermann, B. and Cheresh, D.A., *Current Opinion in Cell Biology,* 5:864-868 (1993)), and Mac-1 (also referred to as CR3, CD11b/CD18, $\alpha_M\beta_2,$ which is expressed on activated leukocytes, such as monocytes and granulocytes (Altieri, D.C. and Edgington, T.S., *Journal of Immunology* 141:2656-2660 (1988)) has been used to prevent the abrupt closure of coronary arteries and to diminish ischemic complications following a coronary artery intervention procedure. However, therapy to mitigate or prevent certain other pathological events can require frequent administration of a therapeutic agent over an extended period of time. The efficacy of this type of therapeutic regimen can be unsatisfactory due to patient compliance issues. Additionally, the frequent administration of therapeutic agents (e.g., proteins or peptides) can elicit the production of antibodies that bind the agent. Thus, the patient can develop immune complex-mediated resistance and/or allergy to a particular therapeutic agent.

WO 98/25971 relates to monovalent antibody fragments. The document discloses antibody fragments which have one or more polymer molecules site-specifically attached through a sulphur atom of a cysteine residue located outside of the variable region domain of the antibody. The polymers include synthetic or naturally occurring polymers such as polyalkylenes, polyalkenylenes, polyoxyalkylenes or polysaccharides. Each fragment may be attached to one or more effector or reporter molecules.

WO 98/36778 relates to a sustained drug delivery composition. Conjugates of chimeric 7E3 antibody or Fab fragments thereof, inter alia, are disclosed in combination with a therapeutic agent. The therapeutic agent may be a phospholipid or heparin (a heteropolysaccharide).

WO 90/06133 relates to heterobifunctional antibodies having specificity for platelets and thrombolytic agents. The document discloses a method of thrombolytic therapy comprising administering to a patient having a thrombus, or at risk of thrombus formation, a heterobifunctional antibody comprising two chemically crosslinked Fab' antibody fragments, the first Fab' fragment being specific for platelet glycoprotein IIb/IIIa receptor complex and the second Fab' fragment being specific for tissue plasminogen activator.

WO 90/11783 relates to platelet specific immunoconjugates. In this document an immunoconjugate comprising a thrombolytic agent and 7E3 is disclosed.

WO 96/40250 relates to a platelet specific chimeric immunoglobulin. The document provides an extensive overview of the potential of 7E3, chimeric 7E3 and corresponding Fab fragments, inter alia, for the inhibition of thrombosis or stenosis in a variety of situations (e.g., following a vascular intervention procedure).

WO 98/32466 relates to a pegylation process. The document discloses general principles of pegylation of antibodies and suggests use of this technique to increase their half-life.

9. Int. Congress of Immunology, 1995, p885, abstract 5247 discloses that the immunogenicity of a murine anti-ICAM-1 monoclonal antibody was reduced 10-fold by the addition of PEG and that binding affinity of the PEGylated antibody for ICAM-1 was decreased.

There is a continuing need for an integrin receptor antagonist with an extended duration of action and, preferably, low immunogenicity.

SUMMARY OF THE INVENTION

The invention relates to modified therapeutic antibodies. The modification serves to improve the pharmacokinetic properties (e.g., increased in vivo serum half-life) without significantly affecting the antigen-binding properties (e.g. affinity) of the antibodies.

In this regard, the invention provides a modified antibody or modified antigen-binding fragment that specifically binds to one or more antigens selected from the group consisting of GPIIb/IIIa, $\alpha_v\beta_3$ and Mac-1, wherein said modified antibody or antigen-binding fragment comprises one to six organic moieties, which are covalently bonded to said antibody or antigen-binding fragment, and which are each, independently, a substituted hydrophilic polymeric group that is substituted with a lipid or phospholipid. In particular embodiments, the invention provides modified antigen-binding fragments of antibodies (e.g., Fv, Fab, Fab', F(ab')$_2$). In a preferred embodiment, the modified antibodies bind to GPIIb/IIIa and/or $\alpha_v\beta_3$ and antagonize the binding of fibrinogen to GPIIb/IIIa and platelet aggregation. In additional embodiments, the binding of the modified antibody of the invention to GPIIb/IIIa and/or $\alpha_v\beta_3$ is completely inhibited by 7E3. Preferably the modified antibody of the invention is 7E3 or a humanized 7E3. A preferred humanized 7E3 is c7E3.

In particular embodiments, the hydrophilic polymeric group can have a molecular weight of about 800 to about 120,000 Daltons and can be a polyalkane glycol (e.g., polyethylene glycol (PEG), polypropylene glycol (PPG)), carbohydrate polymer, amino acid polymer or polyvinyl pyrolidone.

The organic moieties are covalently bonded to a carboxyl-terminus of the antibody and/or covalently bonded to the sulphur atom of a cysteinyl residue of the antibody. Thus, the invention provides antibodies comprising site-specific modifications. For example, a modified Fab of an IgG can comprise a PEG moiety, which is bonded to the carboxyl-terminus of the heavy chain.

In a particular embodiment, a modified c7E3 Fab' comprises two PEG moieties which are each bonded to the sulphur atom of one of the cysteinyl residues that are contained within the hinge region of the heavy chain (the cysteine residues in the hinge region which form inter-chain disulfide bonds in the corresponding IgG or F(ab')$_2$).

In another particular embodiment, a modified c7E3 Fab generated through the action of achromopeptidase, comprises one PEG moiety which is bonded to the carboxyl-terminus of the heavy chain.

The invention also provides use of the products mentioned above in the manufacture of a medicament for treating or preventing diseases and/or disorders in which cells expressing GPIIb/IIIa, $\alpha_v\beta_3$ and/or Mac-1 (e.g., platelets, endothelial cells, leukocytes) play a pathophysiological role. Such diseases and disorders include thromboembolic disorders including cardiovascular disease (e.g., atherosclerosis, angina, coronary artery disease), stroke, ischemia, and tumor vascularization, tumor metastasis and inflammatory conditions. An effective amount of a modified antibody of the invention can be administered to a human in need thereof. An antibody of the invention can be administered alone or in combination with an effective amount of one or more additional active agents.

The modified antibody of the invention can be used in a method of inhibiting platelet-mediated metastasis in a human comprising administering an effective amount of a modified antibody of the invention to the human.

In additional embodiments, the invention provides an *in vitro* method of inhibiting a process (i.e., one or more) mediated through the binding of a ligand to GPIIb/IIIa $\alpha_v\beta_3$ or Mac-1 expressed on the plasma membrane of a cell comprising contacting the cell with an effective amount of a modified antibody or antigen-binding fragment of the invention. GPIIb/IIIa is expressed specifically on platelets and binds several ligands (e.g., fibrinogen, fibronectin, von Willebrand factor, thrombospondin, vitronectin). The $\alpha_v\beta_3$ vitronectin receptor is expressed on cells such as endothelial cells and vascular smooth muscle cells, and to a lesser extent, on platelets, and mediates adhesion to a variety of extracellular matrix proteins (e.g., vitronectin, fibronectin, von Willebrand Factor, fibrinogen, osteopontin, thrombospondin, collagen, perlecan). Mac-1 is expressed on activated leukocytes, such as monocytes and granulocytes, and binds a variety of ligands (e.g., C3bi, Factor X, fibrinogen).

The present invention further relates to a modified antibody or fragment as described herein for use in therapy (including prophylaxis) or diagnosis, and to the use of such a modified antibody or fragment for the manufacture of a medicament for the treatment of a particular disease or condition as described herein (e.g., a thromboembolic disorder, a disease or disorder in which cells expressing GPIIb/IIIa and/or $\alpha_v\beta_3$ play a pathophysiological role).

BRIEF DESCRIPTION OF THE DRAWINGS

Figure 1 is a graph showing dose dependent inhibition of ADP-induced (15 $\mu$M ADP) platelet aggregation (234,000/$\mu$L) by c7E3 Fab and c7E3 Fab'(PEG$_{5000}$)$_2$.

Figure 2 is a graph showing dose dependent inhibition of ADP-induced (20 $\mu$M ADP) platelet aggregation (250,000/$\mu$L) by c7E3 Fab and c7E3 Fab'(PEG$_{20,000}$)$_2$.

Figure 3 is a graph showing that $^{125}$I-c7E3 Fab competitively inhibits binding of c7E3 Fab and c7E3 Fab'(PEG$_{20,000}$)$_2$ to GPIIb/IIIa on GPIIb/IIIa-coated plates.

Figure 4 is a graph showing that $^{125}$I-c7E3 Fab competitively inhibits binding of c7E3 Fab and c7E3 Fab'(PEG$_{20,000}$)$_2$ to $\alpha_v\beta_3$ on $\alpha_v\beta_3$-coated plates.

Figure 5 is a graph showing that $^{125}$I-c7E3 Fab competitively inhibits binding of c7E3 Fab and c7E3 Fab'(PEG$_{5000}$)$_2$ to $\alpha_v\beta_3$ on $\alpha_v\beta_3$-coated plates.

Figure 6 is a graph showing that $^{125}$I-c7E3 Fab competitively inhibits binding of c7E3 Fab and c7E3 Fab'(PEG$_{5000}$)$_2$ to GPIIb/IIIa on GPIIb/IIIa-coated plates.

Figure 7 is a graph showing that $^{125}$I-c7E3 Fab competitively inhibits binding of c7E3 Fab and c7E3 Fab-PEG$_{5000}$ to GPIIb/IIIa on GPIIb/IIIa-coated plates.

Figure 8 is a graph showing that $^{125}$I-c7E3 Fab competitively inhibits binding of c7E3 Fab and c7E3 Fab-PEG$_{5000}$ to $\alpha_v\beta_3$ on $\alpha_v\beta_3$-coated plates.

Figure 9 is a graph showing the rate of clearance of c7E3 Fab'(PEG$_{5000}$)$_2$ and c7E3 Fab'(NEM)$_2$ from the serum of mice.

Figure 10 is a graph showing the rate of clearance of c7E3 Fab'-(PEG$_{20,000}$)$_2$ from the serum of mice.

Figure 11 is a graph showing the rate of clearance of c7E3 Fab' (PEG$_{5000}$)$_2$ and c7E3 Fab from the serum of rats.

Figure 12 is a bar graph showing the titre of specific IgG antibody (anti-c7E3 Fab) produced by mice immunized with c7E3 Fab or c7E3 Fab' (PEG$_{5000}$)$_2$.

Figure 13 is a graph showing dose dependent inhibition of platelet aggregation by c7E3 Fab, c7E3 Fab' (PEG$_{2,000}$)$_2$ or c7E3 Fab'((PEG$_{5,000}$)$_2$)$_2$.

Figure 14 is a graph showing dose dependent inhibition of platelet aggregation by c7E3 Fab or c7E3 Fab'((PEG$_{2,000}$)$_4$)$_2$.

Figure 15 is a graph showing dose dependent inhibition of platelet aggregation by c7E3 Fab or c7E3 Fab'((PEG$_{2,000}$)$_2$)$_2$.

DETAILED DESCRIPTION OF THE INVENTION

The present invention relates to a modified antibody or modified antigen-binding fragment that specifically binds to one or more antigens selected from the group consisting of GPIIb/IIIa, $\alpha_v\beta_3$ and Mac-1, wherein said modified antibody or antigen-binding fragment comprises one to six organic moieties, which are covalently bonded to said antibody or antigen-binding fragment, and which are each, independently, a substituted hydrophilic polymeric group that is substituted with a lipid or phospholipid. In a preferred embodiment, the modified antibody specifically binds to GPIIb/IIIa expressed on the plasma membrane of platelets, thereby inhibiting the binding of fibrinogen to GPIIb/IIIa and platelet aggregation. Preferably, the antibody which binds GPIIb/IIIa further specifically binds the human integrin receptor $\alpha_v\beta_3$.

The antibodies can be polyclonal or monoclonal, and the term "antibody" is intended to encompass both polyclonal and monoclonal antibodies. The terms polyclonal and monoclonal refer to the degree of homogeneity of an antibody preparation, and are not intended to be limited to particular methods of production. The term "antibody" as used herein also encompasses functional fragments of antibodies, including fragments of chimeric, humanized, primatized, veneered or single chain antibodies. Functional fragments include antigen binding fragments which bind to an integrin selected from the group consisting of GPIIb/IIIa, $\alpha_v\beta_3$, and Mac-1. For example, antibody fragments capable of binding to GPIIb/IIIa and/or $\alpha_v\beta_3$ or portions thereof, including, but not limited to Fv, Fab, Fab' and F(ab')$_2$ fragments are encompassed by the invention. Such fragments can be produced by enzymatic cleavage or by recombinant techniques, for example, papain or pepsin cleavage can generate Fab or F(ab')$_2$ fragments, respectively. Other proteases with the requisite substrate specificity can also be used to generate Fab or F(ab')$_2$ fragments. In a preferred embodiment, Fab fragments are prepared by achromopeptidase cleavage. Antibodies can also be produced in a variety of truncated forms using antibody genes in which one or more stop codons has been introduced upstream of the natural stop site. For example, a chimeric gene encoding a F(ab')$_2$ heavy chain portion can be designed to include DNA sequences encoding the CH$_1$ domain and hinge region of the heavy chain. Single chain antibodies, and chimeric, humanized or primatized (CDR-grafted), or veneered antibodies, as well as chimeric, CDR-grafted or veneered single chain antibodies, comprising portions derived from different species, and the like are also encompassed by the present invention and the term "antibody". The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., Cabilly *et al.*, U.S. Patent No. 4,816,567; Cabilly *et al.*, European Patent No. 0,125,023 B1; *Boss et al.*, U.S. Patent No. 4,816,397; Boss *et al.*, European Patent No. 0,120,694 B1; Neuberger, M.S. *et al.*, WO 86/01533; Neuberger, M.S. *et al.*, European Patent No. 0,194,276 B1; Winter, U.S. Patent No. 5,225,539; Winter, European Patent No. 0,239,400 B1; Queen *et al.*, European Patent No. 0 451 216 B1; and Padlan, E.A. *et al.*, EP 0 519 596 A1. See also, Newman, R. *et al.*, *BioTechnology, 10:* 1455-1460 (1992), regarding primatized antibody, and Ladner *et al.,* U.S. Patent No. 4,946,778 and Bird, R.E. *et al.*, *Science, 242:* 423-426 (1988)) regarding single chain antibodies.

As used herein, the term "humanized antibody" refers to an antibody or antigen binding fragment thereof comprising portions of immunoglobulins of different origin, wherein at least one portion is of human origin. For instance, a humanized antibody can comprise an antigen-binding region of nonhuman origin (e.g., rodent) and a constant region of human origin (e.g., a human framework region, a human constant region (e.g., $C_L$, $C_H1$, hinge, $C_H2$, $C_H3$, $C_H4$)). The antigen binding region can be derived from an immunoglobulin of nonhuman origin having the requisite specificity.

In one embodiment, the humanized antibody comprises one or more immunoglobulin chains, comprising a CDR of nonhuman origin (e.g., one or more CDRs derived from an antibody of nonhuman origin) and a framework region derived from a light or heavy chain of human origin (e.g., CDR-grafted antibodies with or without framework changes, including single chain antibodies). In another embodiment, the humanized antibody comprises (a) at least one humanized light chain comprising an antigen binding region derived from the light chain of a nonhuman immunoglobulin of the requisite specificity, and a human light chain constant region, and (b) at least one humanized heavy chain comprising an antigen binding region derived from the heavy chain of said nonhuman immunoglobulin, and a human heavy chain constant region. In one aspect of this embodiment, the humanized antibody is a chimeric antibody, comprising a light chain variable region and a heavy chain variable region of an antibody of nonhuman origin, and a human constant region.

In a preferred embodiment, the humanized antibody can compete with murine 7E3 monoclonal antibody or chimeric 7E3, or an antigen binding fragment thereof, for binding to human GPIIb/IIIa or human $\alpha_v\beta_3$. For example, the antigen-binding region of the humanized antibody can be derived from 7E3 monoclonal antibody. In one aspect of this embodiment, the humanized immunoglobulin comprises the light and heavy chain variable regions of the murine 7E3 antibody, and a human constant region. In another aspect, the humanized immunoglobulin comprises CDR1, CDR2 and CDR3 of the light chain and CDR1, CDR2 and CDR3 of the heavy chain of the murine 7E3 antibody, and a human constant region.

As used herein, the term "chimeric antibody" also refers to an antibody or antigen binding fragment thereof comprising portions of immunoglobulins of different origin. None of the portions of immunoglobulins which comprise a chimeric antibody need to be of human origin. For example, a chimeric antibody can comprise an antigen-binding region of nonhuman origin (e.g., rodent) and a constant region of non-human primate origin (e.g., a chimpanzee framework region, a chimpanzee constant region (e.g., $C_L$, $C_H2$, hinge, $C_H2$, $C_H3$, $C_H4$)).

A humanized or chimeric antibody can comprise a light chain constant region (e.g., $C_K$, $C_\lambda$), and a heavy chain constant region (e.g., $C_H1$, hinge, $C_H2$, $C_H3$, $C_H4$) of a desired isotype, for instance, IgG (e.g., IgG1, IgG2, IgG3, IgG4), IgA (e.g., IgA1, IgA2), IgM , IgE or IgD. In a particular embodiment, a humanized or chimeric antibody can comprise a heavy chain constant region of mixed isotype (e.g., $C_H1$ and hinge of IgG origin and $C_H2$ and $C_H3$ of IgA origin). Preferably, a humanized or chimeric antibody comprises an IgG heavy chain constant region. Most preferably, a humanized or chimeric antibody comprises an IgG1 heavy chain constant region.

Humanized antibodies can be produced using synthetic or recombinant DNA technology using standard methods or other suitable techniques. Nucleic acid (e.g., cDNA) sequences coding for humanized variable regions can also be constructed using PCR mutagenesis methods to alter DNA sequences encoding a human or humanized chain, such as a DNA template from a previously humanized variable region (see e.g., Kamman, *M., et al., Nucl. Acids Res., 17*: 5404 (1989)); Sato, K., *et al., Cancer Research, 53:* 851-856 (1993); Daugherty, B.L. *et al., Nucleic Acids Res., 19(9):* 2471-2476 (1991); and Lewis, A.P. and J.S. Crowe, *Gene, 101:* 297-302 (1991)). Using these or other suitable methods, variants can also be readily produced. In one embodiment, cloned variable regions can be mutagenized, and sequences encoding variants with the desired specificity can be selected (e.g., from a phage library; see e.g., Krebber *et al.*, U.S. 5,514,548; Hoogenboom *et al.*, WO 93/06213, published April 1, 1993)).

Antibodies which are specific for human GPIIb/IIIa, $\alpha_v\beta_3$ or Mac-1 can be raised against an appropriate immunogen, such as isolated and/or recombinant GPIIb/IIIa, $\alpha_v\beta_3$ or Mac-1 or portions thereof (including synthetic molecules, such as synthetic peptides). Antibodies can also be raised by immunizing a suitable host (e.g., mouse) with cells that express GPIIb/IIIa, such as platelets (see e.g., Coller, U.S. Pat. No. 5,440,020) or cells that express $\alpha_v\beta_3$ or Mac-1. In addition, cells expressing recombinant GPIIb/IIIa, $\alpha_v\beta_3$ or Mac-1 such as transfected cells, can be used as immunogens or in a screen for antibody which binds receptor (See e.g., Chuntharapai *et al., J. Immunol.*, 152: 1783-1789 (1994); Chuntharapai *et al.*, U.S. Patent No. 5,440,021).

Preparation of immunizing antigen, and polyclonal and monoclonal antibody production can be performed using any suitable technique. A variety of methods have been described (see e.g., Kohler *et al., Nature,* 256: 495-497 (1975) AND *Eur. J. Immunol.* 6:511-519 (1976); Milstein *et al., Nature* 266: 550-552 (1977); Koprowski *et al.,* U.S. Patent No. 4,172,124; Harlow, E. and D. Lane, 1988, *Antibodies: A Laboratory Manual,* (Cold Spring Harbor Laboratory: Cold Spring Harbor, NY); *Current Protocols in Molecular Biology,* Vol 2 (Supplement 27, Summer '94), Ausubel, F.M. *et al.,* Eds., (John Wiley & Sons: New York, NY), Chapter 11, (1991)). Generally, a hybridoma is produced by fusing a suitable immortal cell line (e.g., a myeloma cell line such as SP2/0 or P3X63Ag8.653) with antibody producing cells. The antibody producing cells, preferably those of the spleen or lymph nodes, can be obtained from animals immunized with the antigen of interest. The fused cells (hybridomas) can be isolated using selective culture conditions, and cloned by limiting dilution. Cells which produce antibodies with the desired specificity can be selected by a suitable assay (e.g., ELISA).

Other suitable methods of producing or isolating antibodies of the requisite specificity can be used, including, for example, methods which select recombinant antibody from a library (e.g., a phage display library), or which rely upon immunization of transgenic animals (e.g., mice) capable of producing a repertoire of human antibodies (see e.g., Jakobovits *et al., Proc. Natl. Acad Sci. USA,* 90: 2551-2555 (1993); Jakobovits *et al., Nature,* 362: 255-258 (1993); Lonberg *et al.,* U.S. Patent No. 5,545,806; Surani *et al.,* U.S. Patent No. 5,545,807; Lonberg *et al.,* WO97/13852).

As used herein the term "specific antibody" or "specific" when referring to an antibody-antigen interaction is used to indicate that the antibody binds a particular antigen with high affinity (e.g., $K_D$ less than about $10^{-5}$ M), rather than to indicate that the antibody binds to only one antigen.

An antibody that binds to GPIIb/IIIa, $\alpha_v\beta_3$ and/or Mac-1, and preferably inhibits the binding of fibronectin to GPIIb/IIIa and inhibits platelet aggregation can be modified as described herein. In one embodiment, 7E3 (a murine IgG1, K) or a humanized 7E3 is modified as described herein. A preferred humanized 7E3 is c7E3, which comprises the antigen binding regions of the heavy and light chains of murine 7E3, and human $\gamma$1 and K constant regions (Knight, D.M.*, et al., Molecular Immunology,* 32(16): 1271-1281 (1995)). Other humanized 7E3 antibodies which can comprise other constant regions of human origin and/or human framework regions can also be modified as described herein. In preferred embodiments, functional fragments (e.g., Fv, Fab, Fab', F(ab')$_2$) of 7E3 or a humanized 7E3 are modified. Other antibodies having an epitopic specificity similar to that of 7E3 or c7E3 for GPIIb/IIIa, $\alpha_v\beta_3$ and/or Mac-1, including antibodies which bind to the same or a functionally equivalent epitope on GPIIb/IIIa, $\alpha_v\beta_3$ and/or Mac-1 as is bound by c7E3 or 7E3 can be modified. Antibodies with an epitopic specificity which is the same or similar to that of c7E3 or 7E3 include antibodies which can block the binding of the c7E3 or 7E3 or a suitable antigen binding fragment thereof (e.g., c7E3 Fab, 7E3 Fab) to GPIIb/IIIa, $\alpha_v\beta_3$ and/or Mac-1. In one embodiment, the antibody 7E3 (murine hybridoma 7E3 was deposited on May 30, 1985 at the American Type Culture Collection, 10801 University Blvd., Manassas, VA 20110-2209, USA, and is available under accession number HB 8832) competitively inhibits the binding of the modified antibody of the invention to GPIIb/IIIa and/or $\alpha_v\beta_3$.

The modification of antibodies as described herein improves the pharmacokinetic properties of the antibody. Specifically, the modified antibodies of the invention are characterized by an increased *in vivo* serum half-life. In a preferred embodiment, the modified antibodies are further characterized by decreased immunogenicity compared to the unmodified antibody. Assessments of the serum half-life and immunogenicity of proteins and peptides (e.g. antibodies, modified antibodies) can be performed using any suitable method. For example, a modified protein can be administered to an animal (e.g., by injection), and serum can be obtained from the animal at a specified time after administration (e.g., a minute, hour, day, week, month, etc.). The serum can be assayed for the presence of the modified protein or for antibodies which bind to the modified protein (e.g., by ELISA).

In a preferred embodiment, the modification does not significantly affect the antigen-binding properties of the antibody. The antigen-binding properties of an antibody can be expressed as the antigen-antibody association rate constant ($k_a$), the antigen-antibody dissociation rate constant ($k_d$) and affinity ($K_D$). The parameters can be measured experimentally using any suitable method. (See, for example, *Berzofsky, et al.,* "Antibody-Antigen Interactions", In *Fundamental Immunology,* Paul, W.E., Ed., Raven Press: New York, NY (1984); Kuby, Janis *Immunology,* W.H. Freeman and Company: New York, NY (1992); and the method described herein). A modification that does not significantly affect the antigen-binding properties of an antibody will not change (e.g., increase or decrease) the affinity of the antibody by more than a factor of about 100. Such modified antibodies bind to an antigen with an affinity that is substantially the same as the affinity of the corresponding unmodified antibody. For example, an antibody can have an affinity of $2 \times 10^{-9}$ M and the modified antibody can have an affinity between about $2 \times 10^{-11}$ M and about $2 \times 10^{-7}$ M. In a preferred embodiment, the modification will not change the affinity of the antibody by more than a factor of about ten. The measured affinity of a particular antibody-antigen interaction can vary if measured under different conditions (e.g., salt concentration, pH). Thus, measurements of antigen-binding parameters (e.g., $k_a$, $k_d$, $K_D$) are preferably made with standardized solutions of antibody and antigen, and a standardized buffer, such as the buffer described herein.

The modified antibodies of the invention comprise one to six organic moieties which are covalently bonded, directly or indirectly, to the antibody, thereby increasing the *in vivo* serum half-life of the antibody, and preferably, decreasing the immunogenicity compared to the unmodified antibody. As used herein, the term "organic moiety" refers to a linear or branched hydrophilic polymeric group substituted with a lipid group (e.g. diacylglycerol group, sphingolipid group (e.g., ceramidyl)) or a phosopholipid group (e.g., phosphatidyl ethanolamine group). Preferred lipid and phospholipid groups are represented by Structural Formulas I and II, respectively.

$$-O-CH_2-CH(R)-CH_2-R' \qquad (I)$$

$$-NH-CH_2-CH_2-O-P(O^-)(O)-O-CH_2-CH(R)-CH_2-R' \qquad (II)$$

In Structural Formulas I and II, R and R' are each, independently, -OH or a group represented by -X-Y, wherein -X- is -O-, -O-C(O)-, -S-, -O-S(O)$_2$-O-, -NH-C(O)- or -O-CH=CH-, and Y is a hydrocarbon group containing from about 6 to about 40 carbon atoms, which is saturated or which contains one or more units of unsaturation (e.g., alkyl, alkenyl, alkynyl), provided that both R and R' are not -OH. Preferably, -X- is -O-C(O)-. Thus, suitable lipid and phospholipid groups include, for example, diacylglycerols, ether phospholipids, thioether phospholipids, lysophospholipids and plasmalogens.

Each organic moiety that is bonded to an antibody of the invention can independently be a hydrophilic polymeric group substituted with a lipid group or a phosopholipid group. As used herein, the term "fatty acid" encompasses monocarboxylic acids and di-carboxylic acids.

A "hydrophilic polymeric group", as the term is used herein, refers to an organic polymer which is more soluble in water than in octane. For example, polylysine is more soluble in water than in octane. Thus, an antibody modified by the covalent attachment of polylysine substituted with a lipid or phospholipid is encompassed by the invention. Hydrophilic polymers suitable for modifying antibodies of the invention can be linear or branched and include, for example, polyalkane glycols (e.g., PEG, monomethoxy-polyethylene glycol (mPEG), PEG diamine, PPG and the like), carbohydrates (e.g., dextran, cellulose, oligosaccharides, polysaccharides and the like), polymers of hydrophilic amino acids (e.g., polylysine, polyarginine, polyaspartate and the like), polyalkane oxides (e.g., polyethylene oxide, polypropylene oxide and the like) and polyvinyl pyrolidone. The hydrophilic polymer which modifies the antibody of the invention can have a molecular weight of about 800 to about 150,000 Daltons as a separate molecular entity.

Preferably, the organic moiety has a molecular weight of about 2,000 Daltons or more as a separate molecular entity. For example, PEG$_{5000}$ and PEG$_{20,000}$, wherein the subscript is the average molecular weight of the polymer in Daltons, are particularly preferred hydrophilic polymers. Also preferred are PEG$_{2000}$, PEG$_{3,400}$, PEG$_{10,000}$, PEG$_{30,000}$ and PEG$_{40,000}$ which can be linear or branched. Branched PEGs (e.g., (PEG$_{2,000}$)$_2$, (PEG$_{2,000}$)$_4$, (PEG$_{5,000}$)$_2$) can be prepared, for example, as described by Harris *et al.*, U.S. Patent No. 5,932,462.

The hydrophilic polymeric group is substituted with a lipid or phospholipid group (as described herein, e.g., Formulas I and II). Preferably, the substituted hydrophilic polymeric group is a linear or branched PEG. More preferably, the substituted hydrophilic polymeric group is a linear PEG (e.g., PEG diamine) that is terminally substituted with a lipid or phospholipid group. Hydrophilic polymers that are substituted with a lipid or phospholipid group can be prepared using suitable methods. For example, as described herein (Examples XIV and XV), a modifying agent can be prepared by reacting monoprotected PEG diamine with an activated fatty acid (e.g., palmitoyl chloride). The resulting product can be deprotected, a suitable activating group can be introduced (e.g., maleimido), and the resulting modifying agent can be used to produce a modified Fab' which comprises a PEG that is terminally substituted with a fatty acid group. A variety of other suitable synthetic schemes can be used, for example, a polymer comprising an amine group can be coupled to a carboxylate of the fatty acid or fatty acid ester as described herein, and an activated carboxylate (e.g., activated with N,N'-carbonyl diimidazole) on a fatty acid or fatty acid ester can be coupled to a hydroxyl group on a polymer.

Fatty acids and fatty acid esters suitable for modifying antibodies of the invention can be saturated or can contain one or more units of unsaturation. In a preferred embodiment, the fatty acids and fatty acid esters comprise from about six to about forty carbon atoms or about eight to about forty carbon atoms. Fatty acids which are suitable for modifying antibodies of the invention include, for example, n-dodecanoate (C$_{12}$, laurate), n-tetradecanoate (C$_{14}$, myristate), n-hexadecanoate (C$_{16}$, palmitate), n-octadecanoate (C$_{18}$, stearate), n-eicosanoate (C$_{20}$, arachidate), n-docosanoate (C$_{22}$, behenate), n-triacontanoate (C$_{30}$), n-tetracontanoate (C$_{40}$), *cis*-$\triangle^9$-octadecanoate (C$_{18}$, oleate), all *cis*-$\triangle^{5,8,11,14}$-eicosatetraenoate (C$_{20}$, arachidonate), octanedioic acid, tetradecanedioic acid, octadecanedioic acid, docosanedioic acid, and the like. Suitable fatty acid esters include mono-esters of dicarboxylic acids which comprise a linear or branched lower alkyl group. The lower alkyl group can comprise from one to about twelve, preferably one to about six, carbon atoms. Suitable fatty acid esters for modifying antibodies of the invention include, for example, methyl octadecanoate, ethyl octadecanoate, propyl octadecanoate, butyl dodecanoate, *sec*-butyl dodecanoate, *tert*-butyl dodecanoate, neopentyl tetradecanoate, hexyl tetradecanoate, methyl *cis*-$\Delta^9$-octadecanoate, and the like.

The modification of antibodies as described herein below is preferably performed using one or more modifying agents. A "modifying agent" as the term is used herein, refers to a derivative of a hydrophilic polymer, a lipid or a phospholipid which comprises an activating group. An "activating group" is a chemical moiety or functional group that can, under appropriate conditions, react with a second chemical group thereby forming a covalent bond between the modifying agent and the second chemical group. For example, amine-reactive activating groups include electrophilic groups such as tosylate, mesylate, halo (chloro, bromo, fluoro, iodo), N-hydroxysuccinimidyl esters (NHS), acyl halides and the like. Activating groups which can react with thiols include, for example, maleimide, iodoacetyl, acrylolyl, pyridyl disulfides, 5-thiol-2-nitrobenzoic acid thiol (TNB-thiol), and the like. An aldehyde functional group can be coupled to amine- or hydrazide-containing molecules, and an azide group can react with a trivalent phosphorous group to form phosphoramidate or phosphorimide linkages. Suitable methods to introduce activating groups into molecules are known in the art (see for example, Hermanson, G. T., *Bioconjugate Techniques,* Academic Press: San Diego, CA (1996)). An activating group can be bonded directly to the hydrophilic polymer,

lipid or a phosopholipid or through a linker moiety, for example a $C_1$-$C_{12}$ hydrocarbyl group. As used herein, "hydrocarbyl group" refers to a hydrocarbon chain wherein one or more carbon atoms is optionally replaced by a heteroatom such as oxygen, nitrogen or sulfur. Suitable linker moieties include, for example, tetraethylene glycol, -$(CH_2)_3$-, -NH-$(CH_2)_6$-NH-, -$(CH_2)_2$-NH- and -$CH_2$-O-$CH_2$-$CH_2$-O-$CH_2$-$CH_2$-O-CH-NH-.

Modifying agents which comprise a linker moiety can be produced, for example, by reacting a mono-Boc-alkyldiamine (e.g., mono-Boc-ethylenediamine, mono-Boc-diaminohexane) with a fatty acid in the presence of 1-ethyl-3-(3-dimethylaminopropyl) carbodiimide (EDC) to form an amide bond between the free amine and the fatty acid carboxylate. The Boc protecting group can be removed from the product by treatment with trifluoroacetic acid (TFA) to expose a primary amine which can be coupled to another carboxylate as described, or can be reacted with maleic anhydride and the resulting product cyclized to produce an activated maleimido derivative of the fatty acid. (See, for example, Thompson, *et al.*, WO 92/16221)

The modified antibodies of the invention can be produced by reacting an appropriate antibody (i.e. antibody or suitable antibody fragment, such as Fab') with a modifying agent, as described herein. In one embodiment, the organic moieties can be bonded to the antibody in a non-site specific manner by employing an amine-reactive modifying agent, for example, an NHS ester of PEG. In a specific example, c7E3 Fab was modified by reaction with NHS-PEG$_{5,000}$ in a manner which was not site specific. As shown herein, this type of modification substantially decreased the binding affinity of the Fab for GPIIb/IIIa (Example XXII, Table 6, compare c7E3 Fab and c7E3 Fab Random (PEG$_{5,000}$)). Thus, in preferred embodiments, the modified antibodies can be produced by reacting an appropriate antibody with one or more modifying agents to produce a modified antibody comprising one to about six organic moieties which are bonded to specific sites on the antibody, for example a (i.e., one or more) carboxyl-terminus and/or the side chain sulfur atom of a (i.e., one or more) cysteinyl residue. In particularly preferred embodiments, the modified antibody can comprise one or two linear PEG moieties of greater than 2,000 Daltons which are bonded to specific sites (e.g., cysteinyl residue which forms an inter-chain disulfide bond, carboxyl terminus of the heavy chain) on the antibody.

In embodiments wherein the modified antibody comprises an organic moiety which is bonded to a cysteinyl residue, it is preferred that the organic moiety is bonded to a cysteinyl residue in the hinge region of the heavy chain that forms an inter-chain disulfide bond in the properly folded (i.e., native) antibody. As used herein, the term "inter-chain disulfide bond", refers to a disulfide bond which links two heavy chains in a properly folded (i.e., native) antibody.

Modified antibodies of this type can be prepared by reducing the inter-chain disulfide bonds, thereby producing monovalent antibodies comprising a heavy chain and a light chain. Suitable reducing agents include, for example, 2-mercaptoethanol (2-ME), dithiothreitol (DTT), 2-mercaptoethylamine (2-MEA) and cysteine. Such monovalent antibodies can be isolated and purified by suitable methods (e.g., column chromatography) and the purified antibody can be reacted with a thiol-reactive modifying agent, for example, O-(2-maleimidoethyl)-O'-methylpolyethylene glycol 5000, to produce the modified antibody of the invention. In one example, the modified antibody comprises a (e.g., one or more) PEG moiety which is bonded to the side chain sulfur atom of at least one of the cysteinyl residues contained within the hinge region of the heavy chain that form an inter-chain disulfide bond.

In another example, the modified antibody is a Fab' which comprises an organic moiety which is bonded to the side chain sulfur atom of at least one of the hinge region cysteinyl residues that form an inter-chain disulfide bond.

In another example, the modified antibody is an Fab comprising an organic moiety which is covalently attached to the carboxyl-terminus of the heavy chain. Modified antibodies of this type can be prepared by reacting an Fab generated by papain cleavage of an IgG with, for example, a PEG comprising an amine activating group in the presence of EDC. However, the amine-activated PEG can react with all carboxyl groups on the antibody making it difficult to purify the antibodies comprising a PEG which is specifically bonded to the carboxyl-terminus of the heavy chain.

A preferred method for preparing the modified antibody is to introduce a unique activating group at the carboxyl-terminus of the heavy-chain through reverse proteolysis. "Reverse proteolysis" is a term of the art which refers to the fact that under particular conditions, certain proteases can catalyze the formation of peptide (amide) bonds. For example, a purified Fab generated by the activity of a protease can be mixed with said protease and carbohydrazide under conditions suitable for reverse proteolysis (e.g., 250-fold molar excess of the carbohydrazide relative to the Fab), to produce an Fab comprising a unique hydrazide function at the carboxyl-terminus of the heavy chain.

The hydrazide containing Fab can be modified by reaction with a suitable modifying agent. For example, a Fab can be reacted with a modifying agent comprising an aldehyde functional group to produce a modified antibody comprising an organic moiety that is specifically attached to the carboxyl-terminus of the heavy chain through a hydrazone-linkage.

The hydrazone can be further stabilized by reaction with a suitable reducing agent (e.g., sodium cyanoborohydride).

Various enzymes which can cleave an antibody to produce a desired fragment can be used to introduce a hydrazide function by reverse proteolysis. The conditions for reverse proteolysis are known to those skilled in the art and include a large (e.g., 250-fold) molar excess of carbohydrazide and an extended reaction time. Additionally, the reverse proteolysis reaction may preferentially occur at a pH different from the optimal pH for proteolysis (Fisch *et al.*, *Bioconjugate Chem.*, 3:147-153 (1992); Werlen *et al.*, *Bioconjugate Chem.*, 5:411-417 (1994); Kumaran *et al.*, *Protein Sci.* 6(10):2233-2241 (1997); *Itoh et al.*, *Bioorg. Chem.*, 24(1): 59-68 (1996); Capellas *et al.*, *Biotechnol. Bioeng.*, 56(4):456-463 (1997)). The optimal pH for reverse proteolysis can be determined empirically using standard methods. A preferred enzyme for carboxyl-terminus modification is achromopeptidase.

The carboxyl-terminus of a light chain can also be modified by the introduction of a hydrazide group by reverse proteolysis. In one embodiment, a protease that can cleave the light chain without significantly affecting the antigen-binding characteristics of the antibody is selected and the light chain modified as described.

In another embodiment, an achromopeptidase or other proteolytic cleavage site can be introduced into the light chain of a cloned antibody employing recombinant DNA technology. This type of antibody can be cleaved by achromopeptidase or a suitable enzyme to generate a Fab wherein a hydrazide group can be introduced at the carboxyl-terminus of both the heavy chain and the light chain by reverse proteolysis.

In additional embodiments, recombinant DNA technology can be used to introduce or remove achromopeptidase or other cleavage sites from the heavy chain of a cloned antibody (e.g., by mutagenesis). For example, the naturally occurring achromopeptidase cleavage site can be removed and a new cleavage site introduce at a different position. Thus, achromopeptidase or other suitable enzymes can be used to generate a variety of modified antibodies of the invention. For example, Fv, Fab, F(ab')$_2$ or Fab' fragments comprising an organic moiety which is bonded to the carboxyl-terminus of the heavy chain and/or the carboxyl-terminus of the light chain can be produced. Such fragments can further comprise one or more additional organic moieties which are bonded to the side chain sulfur atoms of cysteinyl residues.

In a particular embodiment, the modified antibody is a c7E3 Fab' comprising two PEG$_{5,000}$ groups, wherein each PEG$_{5,000}$ group is bonded to the side chain sulfur atom of a cysteinyl residue that forms an inter-chain disulfide bond.

In another particular embodiment, the modified antibody is a c7E3 Fab' comprising two PEG$_{20,000}$ groups, wherein each PEG$_{20,000}$ group is bonded to the side chain sulfur atom of a cysteinyl residue that forms an inter-chain disulfide bond.

In another particular embodiment, the modified antibody is a c7E3 Fab comprising a PEG$_{5,000}$ group which is bonded to the carboxyl-terminus of the heavy chain. In a preferred aspect, the Fab is generated by achromopeptidase cleavage.

In another particular embodiment, the modified antibody is a c7E3 Fab comprising a PEG$_{20,000}$ group which is bonded to the carboxyl-terminus of the heavy chain. In a preferred aspect, the Fab is generated by achromopeptidase cleavage.

In another particular embodiment, the modified antibody is a c7E3 Fab' comprising a PEG$_{5,000}$ group and a C$_{22}$ fatty acid group which are each independently bonded to the side chain sulfur atom of a cysteinyl residue that forms an inter-chain disulfide bond.

In another particular embodiment, the modified antibody is a c7E3 Fab comprising a PEG$_{5,000}$ group which is bonded to the carboxyl-terminus of the heavy chain, wherein the PEG$_{5,000}$ bears a C$_{22}$ fatty acid substituent.

In another particular embodiment, the modified antibody is a c7E3 Fab' comprising a (i.e., one or more) PEG$_{2,000}$ group which is bonded to the side chain sulfur atom of a cysteinyl residue that forms an inter-chain disulfide bond in the hinge region.

In another particular embodiment, the modified antibody is a c7E3 Fab' comprising a (i.e., one or more) PEG$_{10,000}$ group which is bonded to the side chain sulfur atom of a cysteinyl residue that forms an inter-chain disulfide bond in the hinge region.

In another particular embodiment, the modified antibody is a c7E3 Fab' comprising a (i.e., one or more) (PEG$_{2,000}$)$_2$ group which is bonded to the side chain sulfur atom of a cysteinyl residue that forms an inter-chain disulfide bond in the hinge region.

In another particular embodiment, the modified antibody is a c7E3 Fab' comprising a (i.e., one or more) (PEG$_{2,000}$)$_4$ group which is bonded to the side chain sulfur atom of a cysteinyl residue that forms an inter-chain disulfide bond in the hinge region.

In another particular embodiment, the modified antibody is a c7E3 Fab' comprising a (i.e., one or more) (PEG$_{5,000}$)$_2$ group which is bonded to the side chain sulfur atom of a cysteinyl residue that forms an inter-chain disulfide bond in the hinge region.

In another particular embodiment, the modified antibody is a c7E3 Fab' comprising a (i.e., one or more) terminally substituted PEG$_{3,400}$ group which is bonded to the side chain sulfur atom of a cysteinyl residue that forms an inter-chain disulfide bond in the hinge region, wherein the terminal substituent is a palmitoyl group.

In still another particular embodiment, the modified antibody is a c7E3 Fab comprising a $C_{22}$ fatty acid group, which is bonded to the carboxyl-terminus of the heavy chain.

In an additional embodiment, an antibody comprising an engineered cysteinyl residue (e.g., a cysteinyl residue that was introduced through recombinant DNA technology) is modified. Antibodies of this type, including, for example, Fv, Fab, Fab', F(ab')2 fragments, can be readily prepared by those of ordinary skill in the art. The antibody comprising an engineered cysteinyl residue can be reacted with a thiol-reactive modifying agent to produce the modified antibody of the invention. The antibody comprising an engineered cysteinyl residue can further comprise an achromopeptidase or other cleavage site and be modified at this site as described.

The site specific modification (e.g., carboxyl-terminal PEGylation) of antibodies as described herein provides several advantages including an increased duration of action. Further, antibodies comprising site specific modifications can unexpectedly be less immunogenic than the corresponding unmodified antibodies. For example, site specific modification of Fab fragments with one or two low molecular weight organic moieties (e.g., $PEG_{5000}$) produces a modified Fab with an increased serum half-life and significantly decreased immunogenicity, that binds to GPIIb/IIIa or $\alpha_v\beta_3$ with an affinity that is substantially the same as the unmodified antibody (see examples IX, XI and XII). The unexpected benefit of a remarkable decrease in immunogenicity as a result of limited modification is likely due to the site-specific nature of the modification described herein.

The modified antibodies of the invention can be purified by employing methodologies which are known to those of skill in the art. Suitable purification methods can include, for example, column chromatography (e.g., ion-exchange, gel filtration, hydrophobic-interaction, affinity), preparative native electrophoresis, precipitation and ultrafiltration. Preferably, the modified antibodies are purified by hydrophobic-interaction chromatography as described herein.

As used herein, the term "pure" or "purified" refers to a preparation wherein the modified antibody of the invention accounts for about 75% of the macromolecules present in the preparation. Preferably the modified antibody accounts for about 90% or 95% of the macromolecules present in the preparation. It is most preferred that the modified antibody accounts for about 99% or essentially all macromolecules present in the preparation.

The modified antibody of the invention can be used in the manufacture of a medicament for treating (e.g., reducing the severity of or preventing) a disease or disorder in which cells expressing GPIIb/IIIa, $\alpha_v\beta_3$ and/or Mac-1 (e.g., platelets, endothelial cells, leukocytes) play a pathophysiological role in a human. Such disorders include, for example, thromboembolic disorders such as cardiovascular disease (e.g., atherosclerosis, coronary artery disease, angina, myocardial infarction), deep vein thrombosis, stroke, ischemia, tumor vascularization (e.g., $\alpha_v\beta_3$-mediated angiogenesis), tumor metastasis (e.g., platelet-mediated metastasis). Cells which express GPIIb/IIIa, $\alpha_v\beta_3$ and/or Mac-1 (e.g., platelets, endothelial cells, leukocytes) also play a pathophysiological role in inflammatory diseases and conditions, which include, for example, autoimmune diseases, adult respiratory distress syndrome, allograft rejection, sepsis, reperfusion injury (e.g., transplantation associated reperfusion injury, injury from reperfusion in stroke or myocardial infarction patients), myocardial infarction, angioplasty and surgery.

The modified antibody of the invention can also be used in the manufacture of a medicament to beneficially inhibit thrombosis, stenosis, restenosis and/or a process mediated by the binding of a ligand to GPIIb/IIIa, $\alpha_v\beta_3$ and/or Mac-1 (e.g., adhesion, aggregation, secretion (e.g., degranulation), proliferation, generation of oxygen radicals) in a human. For example, the modified antibodies find use in a situation where thrombus formation or reformation or stenosis and/or restenosis is to be prevented, such as, during or following a vascular intervention procedure, for example, angiography, angioplasty (e.g., performed by balloon, atherectomy, laser angioplasty or other suitable methods (with or without rotablation and/or stent placement)), coronary artery by-pass surgery, stent placement (e.g., coronary stent), and/or other vascular intervention procedures (e.g., vascular surgery, vascular graft, deployment of a peripheral stent, insertion of a prosthetic valve or vessel (e.g., in autologous, non-autologous or synthetic vessel graft)). In particular, the method can be used to beneficially inhibit thrombosis during or following a coronary artery intervention procedure, such as percutaneous transluminal coronary angioplasty (PTCA).

The modified antibody of the invention can be used in the manufacture of a medicament for a human to prevent or decrease the severity of a disease and/or disorder in which platelets play a pathophysiological role, or to inhibit (reduce or prevent) thrombosis, stenosis, restenosis and/or a process mediated by the binding of a ligand to GPIIb/IIIa, $\alpha_v\beta_3$ and/or Mac-1. For example, the modified antibody can be administered to a human before, during and/or after a vascular intervention procedure (e.g., angioplasty) to prevent abrupt closure of the vessel (reocclusion) and prevent/or reduce acute ischemia.

An "effective amount", as the term is used herein, is a quantity that is sufficient to achieve a desired therapeutic effect (e.g. inhibit thrombus formation, prevent vessel reocclusion, inhibit stenosis and/or restenosis, inhibit inflammation, inhibit $\alpha_v\beta_3$ - mediated angiogenesis).

The modified antibody of the invention can be used in a method of inhibiting thrombosis in a human comprising administering to said human an effective amount of a modified antibody of the invention.

Alternatively, the modified antibody of the invention can be used in a method of inhibiting stenosis and/or restenosis following a vascular intervention procedure (e.g., coronary artery intervention procedure such as PTCA) in a human comprising administering to said human an effective amount of a modified antibody of the invention.

Alternatively, the modified antibody of the invention can be used in a method of reducing or preventing ischemia in a human comprising administering to said human an effective amount of a modified antibody of the invention.

Alternatively, the modified antibody of the invention can be used in a method of inhibiting the growth and/or metastasis of a tumor in a human comprising administering to said human an effective amount of a modified antibody of the invention.

Alternatively, the modified antibody of the invention can be used in a method of inhibiting $\alpha_v\beta_3$-dependent angiogenesis in a human comprising administering an effective amount of a modified antibody of the invention to the human.

Alternatively, the modified antibody of the invention can be used in a method of inhibiting a process mediated by the binding of a ligand to GPIIb/IIIa, $\alpha_v\beta_3$ and/or MAC-I in a human comprising administering to said human a therapeutically effective amount of a modified antibody of the invention.

One or more modified antibodies of the invention can be administered alone or in combination with an effective amount of one or more additional active agents, for example, a fibrinolytic agent (e.g., Retavase), a thrombolytic agent, such as a plasminogen activator (e.g., tissue plasminogen activator, urokinase, streptokinase, recombinant plasminogen activator), anticoagulant (e.g., heparin, hirulog, hirudin, aspirin), or a coumarin anticoagulant (e.g., warfarin, ethyledine dicoumarol). The modified antibodies of the invention can also be administered with a β-adrenergic blocker (e.g., alprenolol, acebutolol, propanolol), calcium channel blocker (e.g., nifedipine, diltiazem, cinnarizine, bencyclane) or vasodilator (e.g., nitroglycerin, amotriphene, erythritol, prenylamine). The modified antibodies of the invention can also be administered in combination with an agent which stimulates the production of nitric oxide (see, for example, Singh *et al.,* U.S. Pat. No. 5,811,437).

A variety of routes of administration are possible. For example, the modified antibodies of the invention can be prepared and administered in the same manner as has been described for other therapeutic antibodies (Faanes *et al.*, U.S. Pat. No. 5,695,760; Coller *et al.*, WO 95/12412). An affective amount of a modified antibody of the invention can be administered parenterally (e.g., intravenously, intraarterially, intramuscularly, subcutaneously), for example, by injection into a blood vessel, muscle or cavity (e.g., peritoneal cavity, thoracic cavity). The modified antibodies can also be administered orally, topically, by inhalation (e.g., intrabronchial, intranasal, oral inhalation or intranasal drops), or rectally. Preferably, the modified antibodies are administered intravenously. The modified antibody can be administered in a single dose, continuous infusion, or in multiple doses and/or infusions (e.g., a bolus dose followed by continuous infusion).

Formulation of an antibody to be administered will vary according to the route of administration selected (e.g., solution, emulsion, capsule). The modified antibodies of the invention can be administered as neutral proteins or as physiologically acceptable salts. By the term "salt" it is meant that the charged groups on the protein (e.g., protonated amines, carboxylates) are associated with an exchangeable ion of opposite charge. For example, positively charged amino groups (e.g., protonated amines) can form acid addition salts with inorganic acids (e.g., hydrochloric acid, phosphoric acid, methane sulfonic acid, and the like) or organic acids (e.g., acetic acid, lactic acid, citric acid and the like), and contain a counterion such as Cl⁻, $CH_3SO_3^-$, acetate, lactate, citrate, and the like. Salts of carboxylates can be formed by reaction with a suitable base such as a hydroxide base (e.g., sodium hydroxide, potassium hydroxide), and contain a counterion such as Na⁺, K⁺, and the like.

The modified antibody can be administered as part of a pharmaceutical composition comprising a modified antibody and a pharmaceutically acceptable vehicle or carrier. Suitable pharmaceutical carriers may contain inert ingredients which do not interact with the modified antibody. Standard pharmaceutical formulation techniques can be employed, such as those described in *Remington's Pharmaceutical Sciences,* Mack Publishing Company, Easton, PA. Suitable pharmaceutical carriers for parenteral administration include, for example, sterile water, physiological saline, bacteriostatic saline (saline containing about 0.9% mg/ml benzyl alcohol), phosphate-buffered saline, Hank's solution, Ringer's-lactate and the like. The antibody formulation may contain additional additives, such as a stabilizer (e.g., Polysorbate 80, USP/NP). Methods for encapsulating compositions (such as in a coating of hard gelatin or cyclodextran) are known in the art (Baker, *et al., Controlled Release ofBiological Active Agents,* John Wiley and Sons, (1986)).

The amount of antibody to be administered to the human can depend on the type and severity of the disease and on the characteristics of the human, such as general health, age, sex and body weight and tolerance to drugs. It can also depend on the degree, severity and type of disease. The skilled artisan will be able to determine appropriate dosages depending on these and other factors. Typically, a therapeutically effective amount of the modified antibody can range from about 0.01 mg/kg to about 100 mg/kg for a bolus and about 0.001 mg/min to about 1 mg/min for a continuous infusion, although larger or smaller quantity can be used.

EXEMPLIFICATION

Example I Synthesis of c7E3 Fab' (PEG$_{5,000}$)$_2$

18 ml of c7E3 F(ab')$_2$ (6 mg/ml in phosphate buffered saline, prepared by pepsin digestion of c7E3 IgG) was treated with 1.8 ml of dithiothreitol (16 mg/ml) and allowed to stand at room temperature for one hour. 1.6 g of ammonium sulfate were dissolved in the solution, and the Fab' solution was applied to a TosoHaas phenyl-5PW column (21.5 mm X 15 cm, 13μm) which had been equilibrated with 0.1 M sodium phosphate pH 7.5 containing 0.6 M ammonium sulfate. The Fab' was eluted by reducing the ammonium sulfate concentration to 0 over 65 minutes with a flow rate of 6 ml/min. Fractions containing the Fab' were pooled and concentrated in an Amicon stirred cell to a final concentration of 2 mg/ml. A total of 74 mg of the Fab' was obtained. The Fab' was then treated with 2 ml of an aqueous solution of O-(2-maleimidoethyl)-O'-methylpolyethylene glycol 5000 (66.5 mg/ml; 20 eq total) (Shearwater Polymers, Huntsville, Al). After two hours 3.2 g of ammonium sulfate were added and dissolved in the Fab' solution, and the solution was applied to the phenyl column equilibrated with 0.1 M sodium phosphate pH 7.5 containing 0.6 M ammonium sulfate. The PEGylated product was eluted in a 30 to 80% gradient of 0.1 M sodium phosphate pH 7.5 over 65 minutes with a flow rate of 6 ml/min. Pure fractions were combined to yield 45 mg of product. The average molecular weight was determined to be 59665 Daltons by mass spectrometry (MALDI-TOF).

Example II Synthesis of c7E3 Fab'(PEG$_{20,000}$)$_2$

7 ml of c7E3 F(ab')$_2$ (5.2 mg/ml in phosphate buffered saline) were treated with 0.7 ml of dithiothreitol (16 mg/ml) and allowed to stand at room temperature for one hour. 0,63 g Ammonium sulfate were dissolved in the solution and the Fab' solution was applied to a TosoHaas phenyl-5PW column (21.5 mm X 15 cm, 13 μm) which had been equilibrated with 0.1 M sodium phosphate pH 7.5 containing 0.6 M ammonium sulfate. The Fab' was eluted by reducing the ammonium sulfate concentration to 0 over 65 minutes with a flow rate of 6 ml/min. Fractions containing the Fab' were pooled and concentrated in an Amicon stirred cell to a final concentration of 2.2 mg/ml. A total of 22 mg of the Fab' was obtained. The Fab' was then treated with 2 ml of an aqueous solution of O-(2-maleimidoethyl)-O'-methylpolyethylene glycol 20000 (57 mg/ml, 13 eq total)(Shearwater Polymers, Huntsville, Al). After two hours, 0.95 g of ammonium sulfate were dissolved in the solution, and the solution was applied to the phenyl column equilibrated with 0.1 M sodium phosphate pH 7.5 containing 0.6 M ammonium sulfate. The PEGylated product was eluted using a 50 to 100% gradient of 0.1 M sodium phosphate pH 7.5 over 65 minutes with a flow rate of 6 ml/min. Pure fractions were combined to yield 13 mg of product. The average molecular weight was determined to be 91078 Daltons by mass spectrometry (MALDI-TOF).

Example III Synthesis of c7E3 Fab-PEG$_{5,000}$

To 5 ml of c7E3 Fab (7.3 mg/ml) prepared by the action of achromopeptidase on c7E3 IgG was added 1.0 g of carbohydrazide. The pH was adjusted to 4.95 by the addition of 300 μl of glacial acetic acid. 10,000 Units of achromopeptidase were added and the resulting solution incubated at ambient temperature for 24 hours. The solution was dialyzed into 10 mM sodium acetate, pH 4.5. 0.875 mg of monomethoxypolyethylene glycol aldehyde (PEG$_{5,000}$ aldehyde, MW = 5,000) (Shearwater Polymers, Huntsville, A1) were added, followed by the addition of 0.3 mg of sodium cyanoborohydride. The reaction was allowed to stand at ambient temperature overnight. Ammonium sulfate was added to make the final solution 0.6 M in ammonium sulfate. A Tosohaas TSK 5PW phenyl column (21.5 mm x 15 cm) was equilibrated with 100 mM sodium phosphate, pH 7.5 containing 0.6 M ammonium sulfate (buffer A). The reaction mixture was injected onto the phenyl column and eluted with a 60 minute linear gradient of buffer A to 100 mM sodium phosphate, pH 7.5 (buffer B) at a flow rate of 6 ml/min. The peak eluting at approximately 58 minutes was collected, concentrated to 5 ml by ultrafiltration at 40 psi with a membrane having a molecular weight exclusion of 10,000 Daltons. The concentrate was dialyzed into phosphate buffered saline (PBS). A small sample was desalted on a PD-10 column, eluting with water. Mass spectral analysis gave a cluster of peaks (due to the molecular weight dispersion of the PEG$_{5,000}$ aldehyde) centered at 52,846 Daltons, corresponding to the expected molecular weight of the c7E3 Fab after the addition of carbohydrazide and PEG$_{5,000}$ aldehyde.

Example IV Inhibition of Platelet Aggregation by c7E3 Fab'(PEG$_{5,000}$)$_2$ and c7E3 Fab'(PEG$_{20,000}$)$_2$

Preparation of Platelet Rich Plasma (PRP) and Platelet Count Adjustment

All studies were performed utilizing platelets obtained from normal, non-medicated human donors. Blood from each donor was drawn via venipuncture into a 60 ml syringe containing 1/100 final dilution of 40% trisodium citrate. PRP was prepared by centrifugation at 600 g for 4 minutes at room temperature. The PRP was carefully removed and platelet poor plasma (PPP) was prepared by centrifugation of the remaining blood at 2500 g for 20 minutes at room temperature. PRP was allowed to rest at room temperature for 20 minutes prior to counting. PRP platelet counts were determined using a Coulter T-890. The platelet counts were adjusted to 200,000- 250,000 platelets per $\mu$l using autologous PPP.

Platelet Aggregation

Platelet aggregation assays were performed using a PAP-4D platelet aggregometer. For each assay, 450 $\mu$l of PRP was added to an aggregometer cuvette containing a stir bar. Serial dilutions of c7E3 Fab (ReoPro®, Centocor, Inc., Malvern, PA), c7E3, c7E3 Fab'(PEG$_{5,000}$)$_2$ and c7E3 Fab(PEG$_{20,000}$)$_2$ starting at 20 $\mu$g/ml (final concentration) or saline (50 $\mu$l) were added to each cuvette and incubated at 37°C for 10 minutes prior to the start of the run. The cuvettes were then transferred to the aggregometer channels and the stir speed was adjusted to 1200 rpm. Baseline aggregations were monitored for one-half to one minute prior to the addition of 15-20 $\mu$M adenosine diphosphate (ADP). Aggregations were monitored for 4 minutes after the addition of agonist, a control sample, treated with saline only, was included in each run to determine maximal agonist induced platelet aggregation.

Inhibition of Aggregation

Inhibition of aggregation is expressed as a percentage of maximum aggregation of agonist stimulated platelets in the absence of c7E3 Fab, c7E3 Fab'(PEG$_{5,000}$)$_2$ or c7E3 Fab'(PEG$_{20,000}$)$_2$. The extent of inhibition of aggregation for each antibody concentration was calculated as follows:

$$\% \text{ Inhibition} = \left[ 1 - \frac{\% \text{ light transmission antibody}}{\% \text{ light transmission saline control}} \right] \times 100$$

The c7E3 Fab, c7E3 Fab'(PEG$_{5,000}$)$_2$ and c7E3 Fab'(PEG$_{20,000}$)$_2$ final concentrations were mathematically converted to molar concentrations and plotted versus % inhibition of aggregation. c7E3 Fab, c7E3 Fab'(PEG$_{5,000}$)$_2$ and c7E3 Fab'(PEG$_{20,000}$)$_2$ samples demonstrated a similar ability to inhibit ADP mediated platelet aggregation to within a two-fold molar IC$_{50}$ concentration.

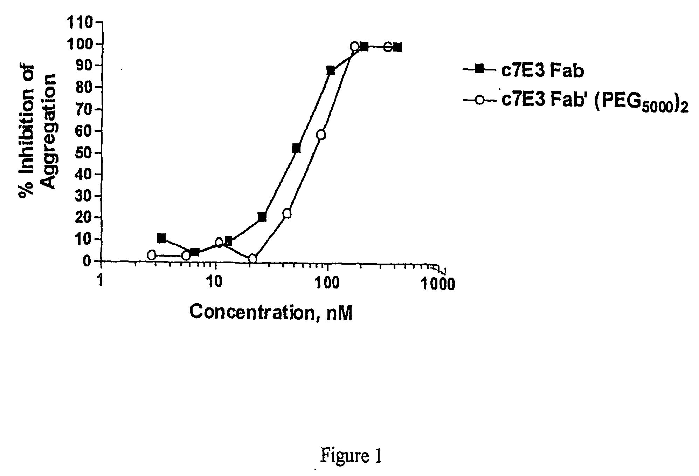

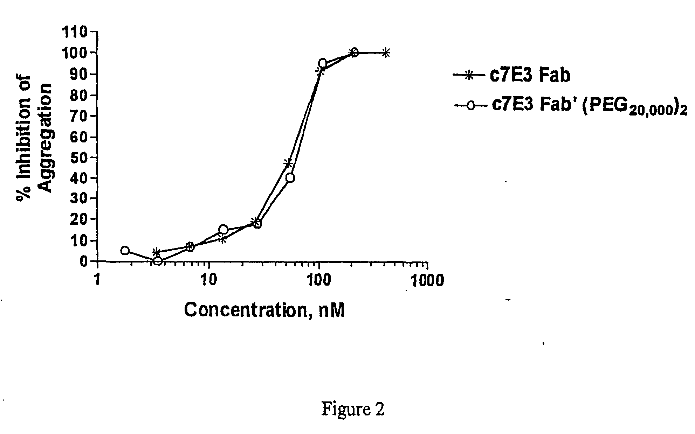

Figures 1 and 2 show that the c7E3 Fab'(PEG$_{5,000}$)$_2$ and c7E3 Fab'(PEG$_{20,000}$)$_2$ are similar to c7E3 Fab in their ability to inhibit platelet aggregation and that the chemical modifications to increase half-life do not affect activity.

Example V Competition binding of c7E3 Fab'-(PEG$_{20,000}$)$_2$ with $^{125}$I-c7E3 Fab to purified integrins

Purified $\alpha_v\beta_3$ (Smith *et al.*, *J. Biol. Chem.* 263(35):18726-18731 (1988)) or GPIIb/IIIa (Pytela *et al., Science* 231(4745):1559-1562 (1986)) was diluted in Tris-buffered saline (TBS) containing 2 mM calcium chloride (TBS/Ca) to a concentration of 0.5 $\mu$g/ml ($\alpha_v\beta_3$) or 1.5 $\mu$g/ml (GPIIb/IIIa). 96-well polystyrene Immulon Removawell plates (Dynex Technologies, Chantilly, VA) *were* coated with $\alpha_v\beta_3$ or GPIIb/IIIa by aliquoting 50$\mu$l of the appropriate protein solution into each well and incubating the plate at room temperature for 2.5 hours. Plates were washed and blocked with 1% bovine serum albumin (BSA) in TBS/Ca for 1 hour at room temperature. c7E3 Fab (ReoPro®, Centocor, Inc., Malvern, PA) was iodinated using Iodobeads® (Pierce Chemicals, Rockford, IL) to approximately 2 $\mu$Ci/$\mu$g. Dilution series of unlabeled c7E3 Fab'-(PEG$_{20,000}$)$_2$ and unlabeled c7E3 Fab were prepared in TBS/BSA/Ca at 2X final concentration. Dilutions of c7E3 Fab'-(PEG$_{20,000}$)$_2$ or c7E3 Fab (starting at 20 $\mu$g/ml final concentration) were mixed 1:1 with 2X $^{125}$I-c7E3 Fab (1 $\mu$g/ml final concentration) and added to plates. The plates were incubated for 2 hours at 37°C. Plates were washed, wells separated into tubes, and bound radioactivity counted in a gamma counter. Data were presented as percent of maximal $^{125}$I-c7E3 Fab bound in the absence of c7E3 Fab'-(PEG$_{20,000}$)$_2$ or c7E3 Fab, and nonlinear regression analysis performed using GraphPad Prism.

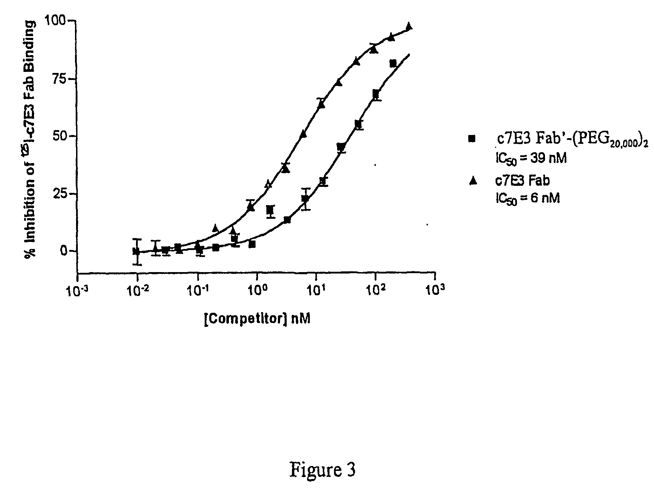

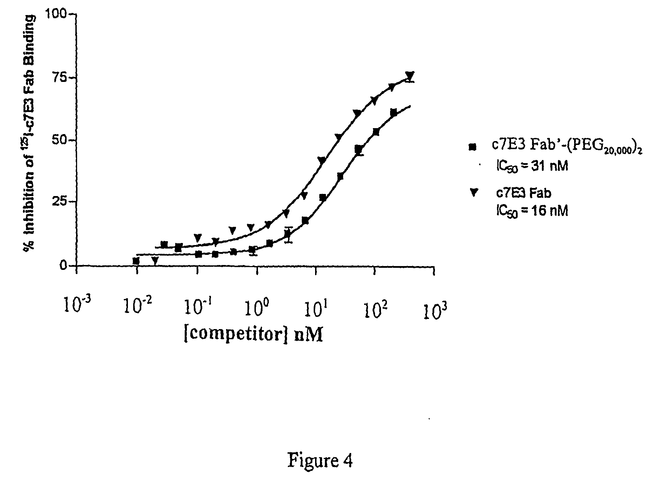

Figures 3 and 4 show the data obtained for both c7E3 Fab and c7E3 Fab'(PEG$_{20,000}$)$_2$ with GPIIb/IIIa and $\alpha_v\beta_3$ respectively, and indicate that the derivatization techniques used to create c7E3 Fab'(PEG$_{20,000}$)$_2$ do not have a significant effect on its binding.

Example VI Competition binding of c7E3 Fab'-(PEG$_{5000}$)$_2$ with $^{125}$I-c7E3 Fab to purified integrins

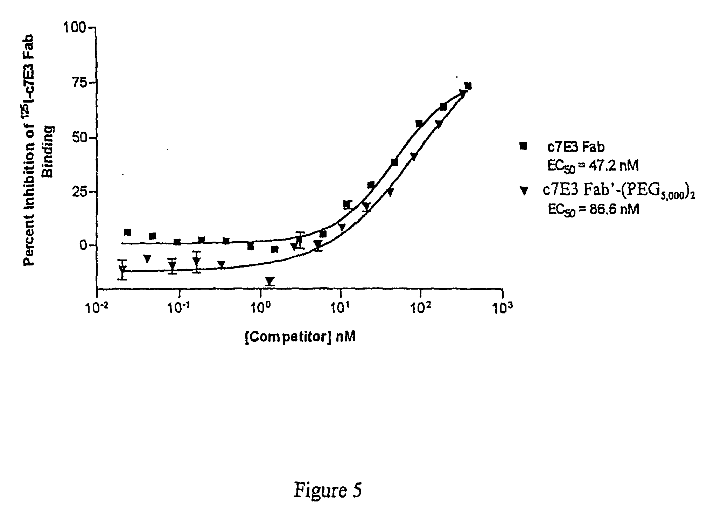

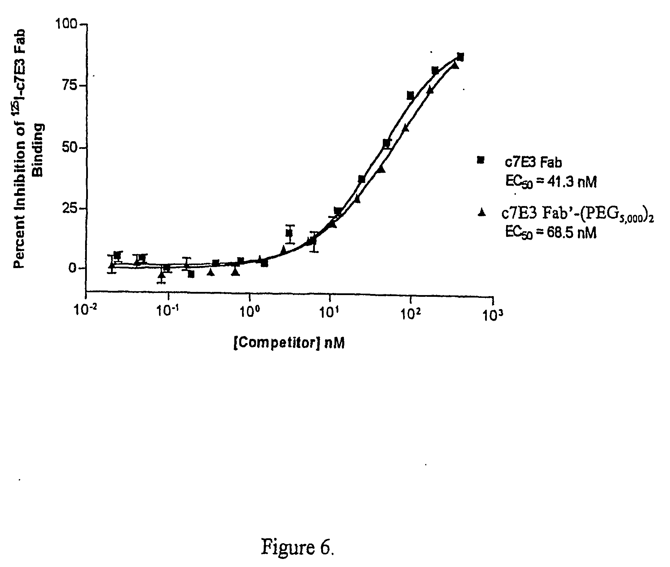

Purified $\alpha_v\beta_3$ (Smith *et al., J. Biol. Chem.* 263(35):18726-18731 (1988)) or GPIIb/IIIa (Pytela *et al., Science* 231(4745):1559-1562 (1986)) was diluted in Tris-buffered saline (TBS) containing 2 mM calcium chloride (TBS/Ca) to a concentration of 0.5 $\mu$g/ml ($\alpha_v\beta_3$) or 1.5 $\mu$g/ml (GPIIb/IIIa). 96 well polystyrene Immulon Removawell plates (Dynex Technologies, Chantilly, VA) were coated with $\alpha_v\beta_3$ or GPIIb/IIIa by aliquoting 60 $\mu$l of the appropriate protein solution into each well and incubating the plates at 4°C overnight. Plates were washed and blocked with 1% bovine serum albumin (BSA) in TBS/Ca for 1 hour at room temperature, Chimeric 7E3 Fab (c7E3 Fab) (ReoPro®, Centocor, Inc., Malvern, PA) was iodinated using Iodobeads® (Pierce Chemicals, Rockford, IL) to approximately 2 $\mu$Ci/$\mu$g. Dilution series of unlabeled c7E3 Fab'-(PEG$_{5000}$)$_2$ and unlabeled c7E3 Fab were prepared in TBS/Ca at 2X final concentration. Dilutions of c7E3 Fab'-(PEG$_{5,000}$)$_2$ or c7E3 Fab (starting at 20 $\mu$g/ml final concentration) were mixed 1:1 with 2X $^{125}$I-c7E3 Fab (1 $\mu$g/ml final concentration) and added to plates. The plates were incubated for 2 hours at 37°C. Plates were washed, wells separated into tubes, and bound radioactivity counted in a gamma counter. Data were presented as percent of maximal $^{125}$I-c7E3 Fab bound in the absence of c7E3 Fab'-(PEG$_{5,000}$)$_2$ or c7E3 Fab, and nonlinear regression analysis performed using GraphPad Prism. Figures 5 and 6 show the data obtained for both c7E3 Fab and c7E3 Fab'(PEG$_{5,000}$)$_2$ with $\alpha_v\beta_3$ and GPIIb/IIIa, respectively, and indicate that the derivatization techniques used to create c7E3 Fab'(PEG$_{5,000}$)$_2$ do not adversely affect the binding.

Example VII Competition binding of c7E3 Fab-PEG$_{5000}$ with $^{125}$I-c7E3 Fab to purified integrins

Purified $\alpha_v\beta_3$ (Smith *et al., J. Biol. Chem.* 263(3S):18726-18731 (1988)) or GPIIb/IIIa (Pytela *et al., Science* 231(4745):1559-1562 (1986)) was diluted in Tris-buffered saline (TBS) containing 2 mM calcium chloride (TBS/Ca) to 0.5 $\mu$g/ml ($\alpha_v\beta_3$) or 1.5 $\mu$g/ml (GPIIb/IIIa). 96 well polystyrene Immulon Removawell plates (Dynex Technologies, Chantilly, VA) were coated with $\alpha_v\beta_3$ or GPIIb/IIIa by aliquoting 60 $\mu$l of the appropriate protein solution into each well and incubating the plates at 4°C overnight. Plates were washed, and blocked with 1% bovine serum albumin (BSA) in TBS/Ca for 1 hour at room temperature. Chimeric 7E3 Fab (c7E3 Fab, ReoPro®, Centocor, Inc., malvern, PA) was iodinated using Iodobeads® (Pierce Chemicals, Rockford, IL) to approximately 2 $\mu$Ci/$\mu$g. Dilution series of unlabeled c7E3 Fab-PEG$_{5000}$ and unlabeled c7E3 Fab were prepared in TBS/Ca at 2X final concentration. Dilutions of c7E3 Fab-PEG$_{5,000}$ or c7E3 Fab (starting at 20 $\mu$g/ml final concentration) were mixed 1:1 with 2X $^{125}$I-c7E3 Fab (1 $\mu$g/ml final concentration) and added to plates. The plates were incubated for 2 hours at 37°C. Plates were washed, wells separated into tubes, and bound radioactivity counted in a gamma counter. Data were presented as percent of maximal $^{125}$I-c7E3 Fab bound in the absence of c7E3 Fab-PEG$_{5,000}$ or c7E3 Fab, and nonlinear regression analysis performed using GraphPad Prism.

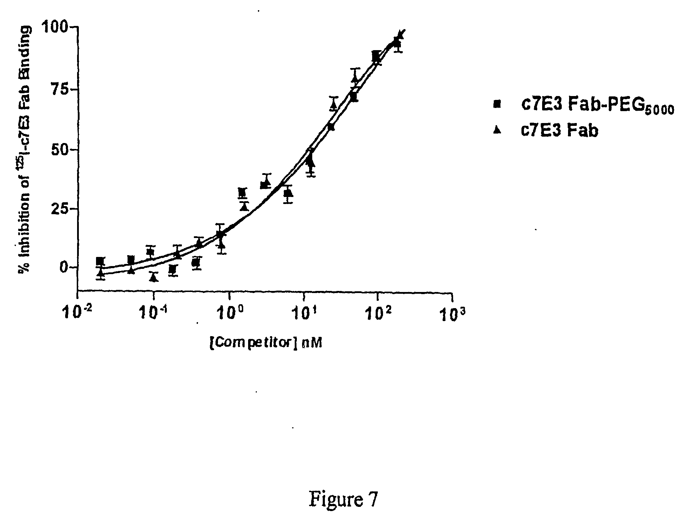

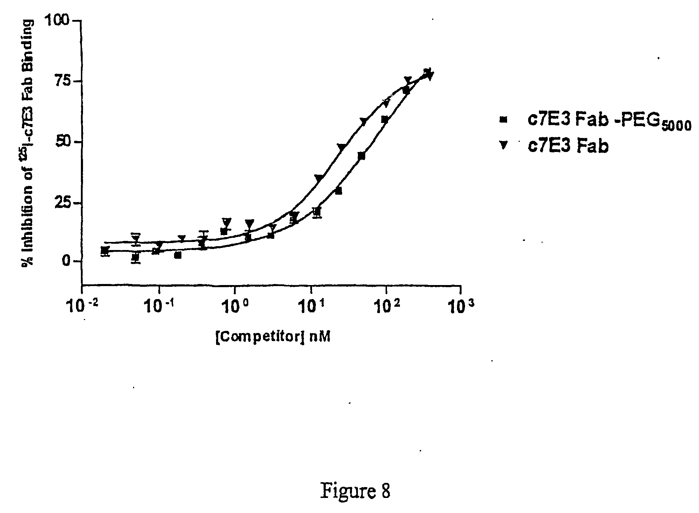

Figures 7 and 8 show the data obtained for both c7E3 Fab and c7E3 Fab-PEG$_{5,000}$ for GPIIb/IIIa and $\alpha_v\beta_3$ ,respectively, and indicate that the derivatization techniques used to create c7E3 Fab-PEG$_{5,000}$ do not adversely affect the binding.

Example VIII GPIIb/IIIa Receptor Blockade (Fibrinogen Bead Assay)

GPIIb/IIIa complexes on activated platelets are receptors for fibrinogen. The mechanism of ligand binding is primarily by Arg-Gly-Asp (RGD) dependent process, whereby peptides containing the RGD sequence block platelet aggregation and inhibit the binding of fibrinogen. It was observed by Coller, *et al.* that platelets will agglutinate fibrinogen-coated beads via the GPIIb/IIIa fibrinogen interaction. Monoclonal antibodies that block the GPIIb/IIIa receptor can prevent the interaction of receptor and ligand and, thus, prevent agglutination of the fibrinogen coated beads.

Covalent Coupling of Fibrinogen to Beads (Carboxylated Polystyrene Microparticles)

Fibrinogen (Enzyme Research, South Bend, IN) was prewarmed to 37°C and reconstituted in warm water. Particulates were removed by centrifuge at 13,000-14,000 g for 7 minutes. Supernatant was removed and its absorbance at OD$_{280}$, E=1.5 was measured. The fibrinogen solution was kept at room temperature until ready for use. The beads (Blue carboxylated polystyrene microparticles 3 $\mu$m diameter, 2.5% solids) were prepared as described (Coller *et al., Circulation* 95(4):860-867 (1997)). Briefly, the beads were brought to room temperature and vortexed vigorously. Beads (5 ml) were placed into a 15 ml centrifuge tube with 0.1 M carbonate buffer, pH 9.6 and centrifuged for 10 minutes at room temp. The pellet was washed once again with carbonate buffer, centrifuged, and the supernatant discarded. The pellet was resuspended in 0.02 M Sodium phosphate buffer, pH 4.5, vortexed vigorously, and centrifuged for 10 minutes at ambient temperature. The supernatant was discarded and the pellet washed two additional times in phosphate buffer. Activation of the beads was accomplished by resuspending the pellet in 6.25 ml of phosphate buffer and slowly adding 6.25 ml of freshly made 2% carbodiimide solution. The bead/carbodiimide solution was mixed for 3 hours on a rocking platform at ambient temperature and then centrifuged for 10 minutes. The supernatant was removed and the pellet was washed three times in phosphate buffer to remove any unreacted carbodiimide. Once the removal of unreacted carbodiimide was complete, the supernatant was removed. At this point the pellet was approximately 250 $\mu$l. The pellet was resuspended in borate buffer to produce a 2% slurry. The 2% bead slurry was cooled in an ice bath with intermittent removal for vortexing to achieve monodispersity. The volume of fibrinogen stock needed to add 0.8 mg fibrinogen per ml of the original 2.5% slurry was calculated. The activated beads were diluted with borate buffer to a concentration that yielded a final reaction density of 1% solids slurry after fibrinogen was added. The beads were coated with fibrinogen overnight with gentle rocking at room temperature. The next day 0.1 ml of 0.25 M ethanolamine per ml of original 2.5% bead slurry was added. The solution was gently mixed for 30 minutes at room temperature to block unreacted sites on the beads. The bead solution was centrifuged for 10 minutes at room temperature. The supernatant was saved for protein determination. To ascertain how well the beads were coated, the amount of fibrinogen that was added originally was subtracted from the amount that was present in the supernatant. The bead pellet was resuspended to a 1% slurry with 10 mg/ml BSA in borate buffer. Again monodispersity was confirmed. The bead/BSA solution was mixed for 30 minutes at room temperature with gentle rocking to block any remaining nonspecific protein binding sites and to wash off loosely bound fibrinogen. The bead/BSA solution was centrifuged for 10 minutes at room temperature. The BSA blocking and centrifugation steps were repeated. The pellet was resuspended to a 2.5% slurry with storage buffer (PBS/1% BSA/0.1%sodium azide/5% glycerol). At this point the beads were stored at 4°C or used in a Fibrinogen Bead Assay.

Assay Setup

70 $\mu$l of blood (1/100 sodium citrate from a human donor having received no medicine for 7 days) and 50 $\mu$l antibody at varying concentrations (diluted in room temperature 10 mM TRIS/0.1% BSA), were mixed and incubated for 10 minutes at room temperature. The blood/antibody mixture was added to a glass test tube 12 x 75 mm containing 20 ocl fibrinogen-coated working beads, 105 $\mu$l 10 mM HEPES buffer, and 20 $\mu$M Thrombin Receptor Activating Peptide (Coller *et al., Circulation* 95(4):860-867 (1997)). The tube was capped and placed on a rocking platform at speed 8 for 4 minutes. Agglutination of beads appeared as moderately sized blue clumps in the blood. As mentioned above, agglutination means that the GPIIb/IIIa receptors are not blocked by the antibody tested.

Fibrinogen Bead Assay with c7E3 Fab'(PEG$_{5,000}$)$_2$ compared to c7E3 Fab

The antibodies were tested in 2-fold dilutions starting at 50 $\mu$g/ml to 0.5 $\mu$g/ml according to the Assay Setup as described above. c7E3 Fab'(PEG$_{5,000}$)$_2$ blocked bead agglutination at concentrations from 108-868 nM while c7E3 Fab (ReoPro®, Centocor, Inc., Malvern, PA) blocked from 131-1050 nM.

Fibrinogen Bead Assay with c7E3 Fab'(PEG$_{20,000}$)$_2$ compared to c7E3 Fab

The antibodies were tested in 2-fold dilutions (from 50 $\mu$g/ml to 0.5 $\mu$g/ml) according to the Assay Setup as described above. c7E3 Fab'(PEG$_{20,000}$)$_2$ blocks GPIIb/IIIa receptors from 143-571 nM while c7E3 Fab (ReoPro®, Centocor, Inc., Malvern, PA) blocks from 263-1050 nM.

Example IX Pharmacokinetics of c7E3 Fab'(PEG$_{5,000}$)$_2$ in mice

On day 0 the study animals (CD-1 female mice weighing 25-30 g) were assigned to one of four treatment groups as shown in Table 1.

Table 1

| Group | N | Test Article | Dose (mg/kg) | Route of admin. | Conc (mg/ml) | Dose Volume (ml/kg) | Post-dose Sample Time Points |
|---|---|---|---|---|---|---|---|
| 1 | 3 | c7E3 Fab'-(NEM)$_2$ | 10 | i.v. | 1.5 | 6.7 | 0.5,2,6 hours |
| 2 | 3 | c7E3 Fab'-(NEM)$_2$ | 10 | i.v. | 1.5 | 6.7 | 1, 4, 8 hours |
| 3 | 3 | c7E3 Fab'-(PEG$_{5,000}$)$_2$ | 10 | i.v. | 1.5 | 6.7 | 6 hours, 2 days, 3 days |
| 4 | 3 | c7E3 Fab'-(PEG$_{5,000}$)$_2$ | 10 | i.v. | 1.5 | 6.7 | 1 hour, 1 day, 4 days, 5 days |

All animals were bled at designated time points. For all groups, 250 $\mu$l of blood for all sample time points (except the final sample) was collected by retro-orbital sinus bleed and the terminal samples were taken by cardiac puncture. Blood was allowed to stand at room temperature for 30-60 minutes, centrifuged at 2500 rpm for 15 minutes and the serum separated and stored at -70°C until pharmacokinetic assays were performed.

Antibody Assay in Mouse Serum

C295A (a Centocor murine monoclonal anti-7E3-idiotype antibody) was diluted in PBS to 5 $\mu$g/ml and coated onto plates (Sterile, 96-well ELISA plates, Corning catalog #25805-96) using 50 $\mu$l/well, and was incubated overnight at 4°C. Plates were then washed 3 times with wash buffer (0.9% sodium chloride, 0.02% Tween-20) using approximately 200 $\mu$l/well per wash. Plates were blocked with a blocking buffer/sample diluent (1% BSA in PBS with 0.1% 2-chloroacetamide) for 1 hour at 37°C, then stored sealed at 4°C and warmed to room temperature before use. Standards were made from 50 $\mu$g/ml stocks which were made from the actual test articles (either c7E3 Fab'-NEM$_2$, or c7E3 Fab'-(PEG$_{5,000}$)$_2$) and using the article which directly corresponded to that used in the animal test group (NEM = N-ethylmaleimide). The standard curve ranged from 1000 ng/ml to 0.95 ng/ml (in 1:2 serial dilutions). Standards were diluted in blocking buffer/sample diluent. Animal serum samples were also diluted in blocking buffer/sample diluent. Five 1:2 serial dilutions of each sample were made with the starting dilution being determined by estimation based on dose and time point. A plate blank of blocking buffer/sample diluent was added to each plate in triplicate at 50 $\mu$l/well. Standards and samples were applied to the plates in triplicate at 50 $\mu$l/well and incubated at 37°C for 1 hour. Plates were then washed as above. Test article captured from the standards/samples by the plated C295A monoclonal antibody was then detected by adding biotinylated C281A monoclonal antibody (a Centocor murine monoclonal anti-7E3 idiotype antibody that does not compete with C295A) diluted in blocking buffer/sample diluent at 50 $\mu$l/well. Plates were incubated at 37°C for 1 hour and then washed as above. Streptavidin-labelled horseradish peroxidase (Amersham, Catalog #RPN.1051) was diluted in blocking buffer/sample diluent and added to the plates at 50 $\mu$l/well and plates were incubated at 37°C for 30 minutes. Ten minutes prior to the end of the incubation step, OPD (o-phenylenediamine) tablets (Sigma, Catalog #P-8412) were dissolved in the OPD diluent (citrate/phosphate buffer with 0.03% hydrogen peroxide and 0.1% 2-chloroacetamide) to I mg/ml. Plates were then washed as above and the OPD substrate was added at 200 $\mu$l/well and allowed to develop, in the dark, at room temperature for 15 minutes. Development was stopped by the addition of 4 N H$_2$SO$_4$ at 50 $\mu$l/well. The OD$_{490}$ was read for each plate and samples evaluated under a four parameter fit of the standards with the plate blank value being subtracted from all wells. The two dilutions closest to the midpoint of the standard curve were averaged for each sample dilution set. Samples which did not dilute out were repeated using either a higher, or lower, initial dilution as appropriate.

Other anti-7E3-idiotype antibodies suitable for use in the described assay can be prepared by known means. For example, a suitable host animal (e.g., mouse) can be immunized with 7E3 and hybridomas can be prepared and screen by any suitable technique, such as those described herein.

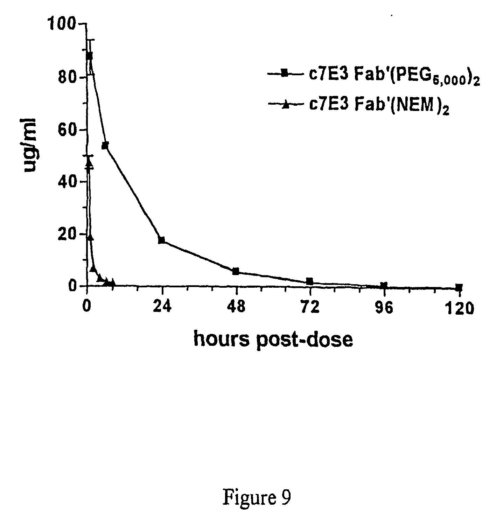

Figure 9 shows the serum levels versus time of the test articles and demonstrates the significantly increased half-life of the c7E3 Fab'-(PEG$_{5,000}$)$_2$ over the NEM-blocked Fab'.

Example X Pharmacokinetics of c7E3 Fab'(PEG$_{20,000}$)$_2$ in mice

On day 0 the study animals (CD-1 female mice weighing 25-30 g) were assigned to one of two treatment groups as shown in Table 2.

Table 2

| Group | n | Test Article | Dose (mg/kg) | Route of admin. | Conc (mg/ml) | Dose Volume (ml/kg) | Post-dose Sample Time Points |
|---|---|---|---|---|---|---|---|
| 1 | 3 | C7E3 Fab'-(PEG$_{20,000}$)$_2$ | 18 | i.v. | 1.3 | 13.8 | 6 hours, 2 days, 3 days |
| 2 | 3 | C7E3 Fab'-(PEG$_{20,000}$)$_2$ | 18 | i.v. | 1.3 | 13.8 | 1 hour, 1 day, 4 days, 5 days |

All animals were bled at designated time points. For all groups, 250 $\mu$L of blood for all sample time points (except the final sample) was collected by retro-orbital sinus and the terminal samples were taken by cardiac puncture. Blood was allowed to stand at room temperature for 30-60 minutes, centrifuged at 2500 rpm for 15 minutes and the serum separated and stored at -70°C until pharmacokinetic assays were performed. Assay for serum levels of c7E3 Fab'-$(PEG_{20,000})_2$ were performed as in Example IX.

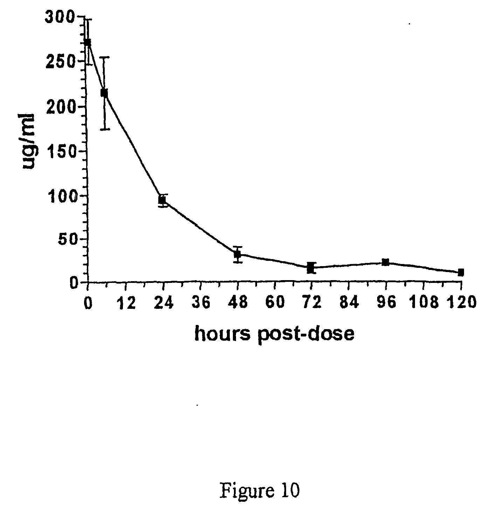

Figure 10 shows the increased persistence in the serum of c7E3 Fab'$(PEG_{20,000})_2$.

Example XI Pharmacokinetics of c7E3 Fab'$(PEG_{5,000})_2$ in rats

Male CD1 rats weighing approximately 350 g were assigned to one of four treatment groups as shown in the Table 3.

Table 3

| Group | N | Test Article | Dose (mg/kg) | Route of Admin. | Test Article (mg/ml) | Dose Volume (ml/kg) | Sample Time Points |
|---|---|---|---|---|---|---|---|
| 1 | 3 | c7E3 Fab | 20 | i.v. | 2.0 | 10 | 1, 10, 60 min |
| 2 | 3 | c7E3 Fab' $(PEG_{5,000})_2$ | 20 | i.v. | 2.0 | 10 | 0, 10, 60 min |
| 3 | 3 | c7E3 Fab' $(PEG_{5,000})_2$ | 20 | i.v. | 2.0 | 10 | 6 hours |
| 4 | 3 | Saline | 20 | i.v. | 0 | 10 | 6 hours |

Animals were individually weighed and the test article dosages calculated. Indwelling intravenous catheters were inserted into the lateral tail veins and taped into place. *Animals were anesthetized with sodium pentobarbital prior to the pre-*dose blood sample and remained anesthetized throughout the entire course of blood sampling. Blood samples were collected according to the schedule in the above table by cardiac puncture using 3 ml syringes containing 16 $\mu$l of 40% sodium citrate. Blood samples were transferred from the syringes to 1.5 ml microfuge tubes and spun at 10,000 rpm briefly (4-5 seconds) in a microcentrifuge. The Platelet Rich Plasma (PRP) was removed from the top and the remainder of the blood sample spun at 10,000 rpm for 5 minutes in a microcentrifuge to obtain Platelet Poor Plasma (PPP). Platelet counts of the PRP samples were determined and the platelet counts adjusted to between 200,000 and 300,000 platelets/$\mu$l with autologous PPP using a Coulter Counter ZM. The remaining PPP was frozen for determination of free Fab or Fab'$(PEG_{5,000})_2$ concentration.

Platelet aggregation was performed on a four-channel Bio/Data PAP4C aggregometer which allowed simultaneous evaluation of the aggregation responses of the pre-dose, 10 minute and 60 minute post-dose PRP samples for each animal. For each sample, 250 $\mu$l of PRP was transferred to a siliconized aggregometer curvette and incubated without stirring at 37° C for 10 minutes. The baseline aggregation signal was monitored for 1 minute. ADP (10 $\mu$M) was added to each cuvette and the aggregation response monitored for an additional 4 minutes. Maximal response (% light transmittance) for each PRP sample was recorded. The percent inhibition of the aggregation of the post-dose PRP samples relative to the pre-dose PRP samples was determined using the following equation:

$$C = (A-B)/A \times 100$$

where:

    C= % inhibition of aggregation of the PRP post-dose sample
    A = % light transmittance of the PRP pre-dose sample
    B = % light transmittance of the PRP post-dose sample

Serum levels of Fab or Fab'$(PEG_{5,000})_2$ were determined as outlined in Example VIII.

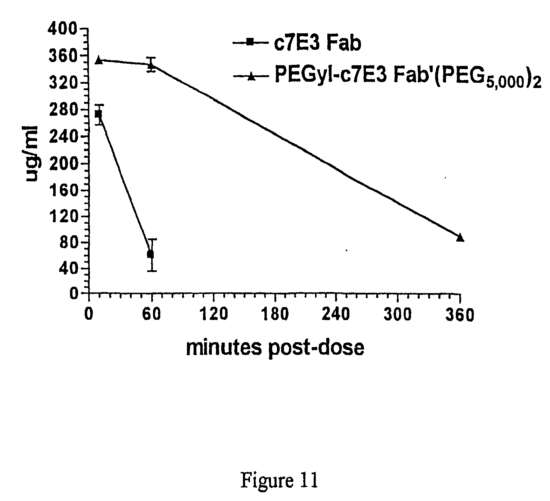

Figure 11 shows that the serum levels of Fab'$(PEG_{5,000})_2$ are more persistent than the corresponding Fab, showing conclusively the extended half-life of the Fab'$(PEG_{5,000})_2$.

That the extended persistence of c7E3 Fab'$(pEG_{5,000})_2$ in serum is reflected in an increased duration of activity is shown by the ex-vivo aggregation data in the following table. Aggregation at 6 hours with c7E3 Fab'$(PEG_{5,000})_2$ is similar to aggregation at 60 minutes with c7E3 Fab.

Table 4

|  |  | % aggregation | |
|  |  |  | |
| Animal # | Test Article | 60 min | 6 hours |
| 1 | c7E3 Fab | 14% | |
| 2 | c7E3 Fab | 28% | |
| 3 | c7E3 Fab | 38% | |
| 4 | c7E3 Fab'(PEG$_{5,000}$)$_2$ | 0% | |
| 5 | c7E3 Fab'(PEG$_{5,000}$)$_2$ | 0% | |
| 6 | c7E3 Fab'(PEG$_{5,000}$)$_2$ | 0% | |
| 7 | c7E3 Fab'(PEG$_{5,000}$)$_2$ | | 18% |
| 8 | c7E3 Fab'(PEG$_{5,000}$)$_2$ | | 29% |
| 9 | c7E3 Fab'(PEG$_{5,000}$)$_2$ | | 26% |

Example XII Determination of the binding kinetics of c7E3 Fab, c7E3 Fab', c7E3 F(ab')$_2$ or their analogs

A goat (Fab')$_2$ anti-human (Fab')$_2$ (Jackson Labs) was covalently immobilized to a BIAcore CM-5 (carboxymethyl) chip using NHS/EDC (N-hydroxysuccinimide/N-ethyl-N'(dimethylaminopropyl) carbodiimide). The chip was activat-ed with 40 $\mu$l of a 1:1 mixture of the coupling reagents obtained from BIAcore and prepared according to the manufacturers instructions at 10 $\mu$l/min. A 20 $\mu$g/ml solution of the (Fab')$_2$ in 10 mM sodium acetate, pH 4.8 was flowed over the chip at 10 $\mu$l/min until 2,500 RU were captured. Unreacted activated carboxyl groups were capped with a 40 $\mu$l injection of 1 M ethanolamine. Using a running buffer of 10 mM tris buffered saline, pH 7.5 containing 2 mM calcium chloride, 2 mM magnesium chloride, 0.01% Tween-100 and 25 $\mu$g/ml BSA, c7E3 Fab, c7E3 Fab' or c7E3 (Fab')$_2$ were flowed over the immobilized anti-human F(ab')$_2$ to capture 500-700 RU. 250 $\mu$l of a solution of GPIIb/IIIa or $\alpha_v\beta_3$ (from 2-10 $\mu$g/ml) were flowed over the capture antibody fragments at 30 $\mu$l/min followed by an uninterrupted dissociation period of 800 seconds. The chip was regenerated with sequential 15 $\mu$l injections of 100 mM phosphoric acid containing 0.05% CHAPS. The association and dissociation phases were analyzed using BIAevaluation 3.0 (BIAcore) for global association ($k_a$) and dissociation ($k_d$) constants. $K_D$ was calculated by dividing $k_a$ by $k_d$. The binding constants shown in Table 5 indicate that the derivatization techniques used to create the c7E3 Fab-PEG$_{5,000}$, c7E3 Fab'-(PEG$_{5,000}$)$_2$, and c7E3 Fab'-(PEG$_{20,000}$)$_2$ do not adversely affect the binding.

Table 5

| Binding constants. | | | |
| --- | --- | --- | --- |
|  | $k_{a(mol^{-1}sec^{-1})}$ | $k_{d(sec^{-1})}$ | $K_D$ (M) |
| c7E3 Fab | 4.55 x 10$^4$ | 5.22 x 10$^{-5}$ | 1.15 x 10$^{-9}$ |
| c7E3 Fab'(PEG$_{5,000}$)$_2$ | 4.87 x 10$^4$ | 8.15 x 10$^{-5}$ | 1.67 x 10$^{-9}$ |
| c7E3 Fab'(PEG$_{20,000}$)$_2$ | 4.48 x 10$^4$ | 10.60 x 10$^{-5}$ | 2.36 x 10$^{-9}$ |
| c7E3 Fab PEG$_{5,000}$ | 1.71 x 10$^4$ | 7.65 x 10$^{-5}$ | 4.47 x 10$^{-9}$ |

Example XIII Immunogenicity of c7E3 Fab'(PEG$_{5,000}$)$_2$ as compared to c7E3 Fab

Animals and Reagents:

Two groups of six week old Balb/c mice from Charles River Laboratories (eight animals per group) were immunized by intra peritoneal (IP) injection with 100 $\mu$g c7E3 Fab (ReoPro®, Centocor, Inc., Malvern, PA) or c7E3 Fab'(PEG$_{5,000}$)$_2$ diluted in physiological saline (Baxter) and emulsified in an equal volume of complete Freund's adjuvant. A subsequent IP injection with 100 $\mu$g c7E3 Fab (ReoPro®, Centocor, Inc., Malvern, PA) or c7E3 Fab'(PEG$_{5,000}$)$_2$ emulsified in incomplete Freund's adjuvant was given 14 days later. The mice were bled by retro-orbital puncture without anti-coagulant on day 20. The blood was allowed to clot at room temperature for one hour and the serum was collected.

The immunogenicity of c7E3 Fab or c7E3 Fab'(PEG$_{5,000}$)$_2$ was evaluated by measuring the immune response via a solid phase enzyme linked immunoassay (EIA). c7E3 Fab was coated on 96-well flat bottom EIA plates at 10 $\mu$g/ml in 10 mM carbonate buffer, pH 9.6, 50 $\mu$L/well overnight at 4°C. After washing in 0.15 M saline and 0.02% (v/v) Tween 20, the wells were blocked with 1% (w/v) bovine serum albumin (BSA) in phosphate buffered saline (PBS), 200 $\mu$L/well for 1 hour at RT. Plates were used immediately or frozen at -20 C for future use.

All sera were diluted 1:50 in PBS and stored at 4°C until tested. Doubling dilutions of immune sera were incubated on the immunogen coated plates at 50 $\mu$L/well at room temperature (RT) for I hour. The plates were washed and then probed with 50 $\mu$L/well goat anti-mouse IgG, Fc specific, horseradish peroxidase labeled conjugate diluted 1:20,000 in 1% BSA-PBS for 1 hour at RT. The plates were again washed and 100 $\mu$L/well of the citrate-phosphate substrate solution [0.1M citric acid and 0.2M sodium phosphate, 0.01 % H$_2$O$_2$ (Sigma) and 1 mg/mL phenylenediamine dihydrochloride] was added for 15 minutes at RT. Stop solution (4 N sulfuric acid) was then added at 25 $\mu$L/well and the optical densities were read at 490 nm on a Dynatech MR 5000 automated plate spectrophotometer.

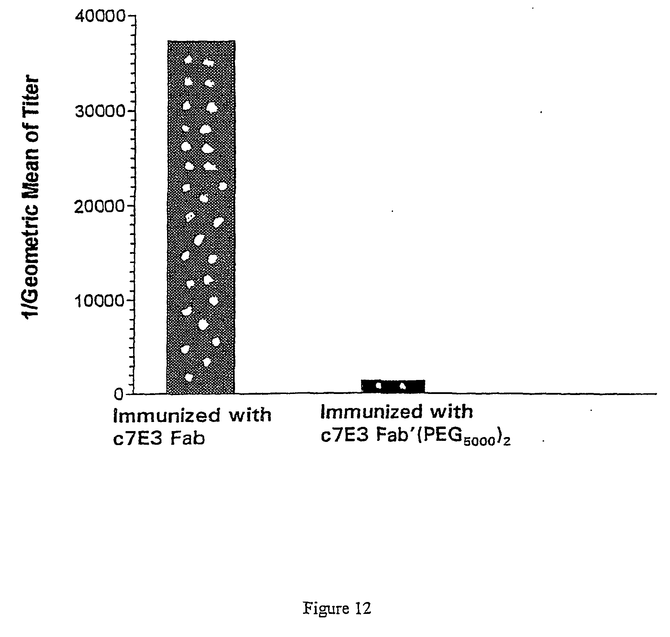

Figure 12 shows the inverse geometric mean of the titers. C7E3 Fab'(PEG$_{5,000}$)$_2$ shows significantly less immunogenicity that c7E3 Fab.

Example XIV Synthesis of N-Maleimidoacetyl-N'-palmitoyl-PEG$_{3,400}$

Step 1

Palmitoyl chloride (80.8 mg, 0.2942 mM) was dissolved in 2 mL acetone and added dropwise to a solution of N-*tert*-butoxycarbonyl-PEG$_{3,400}$ diamine (Shearwater Polymers, Inc.)(500 mg, 0.1471 mM) in 5.0 mL of pyridine and the resulting reaction mixture was refluxed for three hours. The pyridine was evaporated under reduced pressure and the resulting oil was treated with 40 mL of ethyl ether to give a white precipitate that was removed by filtration, washed with ether and dried under reduced pressure to give 554 mg of white solid. The crude material was purified using silica gel column chromatography (Baker Silica Gel, 40$\mu$ m, flash chromatography packing, glass column, 2 x 30 cm) eluting with chloroform : methanol (9:1, v/v). Fractions were pooled, evaporated to dryness and triturated with ethyl ether. A white precipitate was recovered by filtration, washed with ether then dried under reduced pressure to give 266 mg of N-*tert*-butoxycarbonyl-N'-palmitoyl-PEG$_{3,400}$ (MH$^+$ 3770.15 (cluster)).

Step 2

N-*tert*-butoxycarbonyl-N'-palmitoyl-PEG$_{3,400}$ (227 mg) was stirred with 25 mL of trifluoroacetic acid : dichloromethane (1:1, v/v) for 15 minutes at ambient temperature. Solvents were evaporated under reduced pressure and ethyl ether (100 mL) was added to the residue. The resulting white precipitate was recovered by filtration, washed with ether and dried under reduced pressure to yield 141 mg of N-palmitoyl-PEG$_{3,400}$ amine trifluoroacetate.

Step 3

N-palmitoyl-PEG$_{3,400}$ amine trifluoroacetate (350 mg, 0.0925 mM) was dissolved in 3.5 mL tetrahydrofuran and diisopropylethylamine (40 mL) was added to adjust the pH to about 8.0 (as measured with a moistened indicator paper). After about 10 minutes of stirring, maleimido acetic acid N-hydroxysuccinamide ester (48 mg, 0.185 mM) (Molecular Biosciences, Lot 11158) was added and the mixture was stirred for about an additional four hours. The addition of diethyl ether (90 mL) resulted in the precipitation of a white solid that was isolated and dried under reduced pressure to yield 330 mg of N-Maleimidoacetyl-N'-palmitoyl-PEG$_{3,400}$ (MH$^+$ 3806.89 (cluster)).

Example XV Synthesis of c7E3 Fab'(Palmitoyl-PEG$_{3,400}$)$_2$

c7E3 F(ab)'$_2$ (43.6 mg in 9 mL of PBS) was placed in a water bath (37°C). 135 $\mu$L of 1.0 M L-cysteine (Sigma, St. Louis) in water was added and the reaction mixture let stand at 30°C for 30 minutes. Ammonium sulfate (714 mg) was added and the reaction mixture was loaded onto an HPLC column (Tosohaas, Phenyl 5PW, 10 $\mu$m, 0.75 x 7.5 cm) and eluted with a 60 minute linear gradient (0-100%) of 0.1 M sodium phosphate/0.6 M ammonium sulfate/0.05% EDTA, pH 6.5 to 0.1 M sodium phosphate/0.05% EDTA, pH 6.5 at a flow rate of 6 mL per minute. The peak corresponding to c7E3 Fab' was collected. To this was added 28 mg of N-Maleimidoacetyl-N'-pabnitoyl-PEG$_{3,400}$ and the reaction was gently agitated for about 30 minutes to give the titled compound (MH$^+$ 56,362.4 (cluster)).

Example XVI Synthesis of 7E3 Fab'(PEG$_{10,000}$)$_2$

c7E3 F(ab)'$_2$ (28.8 mg in 9.5 mL of PBS) was placed in a water bath (37°C). 182 $\mu$L of 1.0 M L-cysteine (Sigma, St. Louis) in water was added and the reaction mixture let stand at 37°C for 30 minutes. Ammonium sulfate (761 mg) was added and the reaction mixture was loaded onto an HPLC column (Tosohaas, Phenyl 5PW, 10 $\mu$m, 0.75 x 7.5 cm) and eluted with a 60 minute linear gradient (0-100%) of 0.1 M sodium phosphate/0.6 M ammonium sulfate/0.05% EDTA, pH 6.5 to 0.1 M sodium phosphate/0.05% EDTA, pH 6.5 at a flow rate of 6 mL per minute. The peak corresponding to c7E3 Fab' was collected. Methoxy-PEG$_{10,000}$-maleimide (Shearwater polymers, Inc) (46.5 mg) was added to the Fab' solution and gently agitated for 15 hours at ambient temperature. The reaction mixture was concentrated to 10 mL and loaded onto an HPLC column (Tosohaas, Phenyl 5PW, 10 $\mu$m, 0.75 x 7.5 cm) and eluted with a 60 minute linear gradient (0-100%) of 0.1 M sodium phosphate/0.6 M ammonium sulfate, pH 7.5 to 0.1 M sodium phosphate, pH 7.5 at a flow rate of 6 mL per minute. The peak corresponding to the title compound was collected, concentrated and reloaded onto an HPLC column (Tosohaas, Phenyl 5PW, 10 $\mu$m, 0.75 x 7.5 cm) and eluted with a 60 minute linear gradient (50-70%) of 0.1 M sodium phosphate/0.6 M ammonium sulfate, pH 7.5 to 0.1 M sodium phosphate, pH 7.5 at a flow rate of 6 mL per minute. The peak correspond to the titled compound was collected and concentrated to yield 9 mg of product (MH$^+$ 69,832 (cluster)).

Example XVII Synthesis of c7E3Fab'(PEG$_{2,000}$)$_4$)$_2$

c7E3 F(ab)'$_2$ (40.1 mg in 9.5 mL of PBS) was placed in a water bath (37°C). 163 $\mu$L of 1.0 M L-cysteine (Sigma, St. Louis) in water was added and the reaction mixture let stand at 37°C for 30 minutes. Ammonium sulfate (777 mg) was added and the reaction mixture was loaded onto an HPLC column (Tosohaas, Phenyl 5PW, 10 $\mu$m, 0.75 x 7.5 cm) and eluted with a 60 minute linear gradient (0-100%) of 0.1 M sodium phosphate/0.6 M ammonium sulfate/0.05% EDTA, pH 6.5 to 0.1 M sodium phosphate/0.05% EDTA, pH 6.5 at a flow rate of 6 mL per minute. The peak corresponding to c7E3 Fab' was collected. (PEG$_{2,000}$)$_4$-maleimide (Shearwater Polymers, Inc.) (50.2 mg) was added to the Fab' solution and gently agitated for 2 hours at ambient temperature. The reaction mixture was concentrated to 9.8 mL and loaded onto an HPLC column (Tosohaas, Phenyl 5PW, 10 $\mu$m, 0.75 x 7.5 cm) and eluted with a 60 minute linear gradient (0-100%) of 0.1 M sodium phosphate/0.6 M ammonium sulfate, pH 7.5 to 0.1 M sodium phosphate, pH 7.5 at a flow rate of 6 mL per minute. The peak corresponding to the title compound was collected, concentrated and reloaded onto an HPLC column (Tosohaas, Phenyl 5PW, 10 $\mu$m, 0.75 x 7.5 cm) and eluted with a 60 minute linear gradient (50-70%) of 0.1 M sodium phosphate/0.6 M ammonium sulfate, pH 7.5 to 0.1 M sodium phosphate, pH 7.5 at a flow rate of 6 mL per minute. The peak correspond to the titled compound was collected and concentrated to yield 13.5 mg of product (MH$^+$ 65,942 (cluster)).

Example XVIII Synthesis of c7E3Fab'((PEG$_{2,000}$)$_2$)$_2$

c7E3 F(ab)'$_2$ (54.3 mg in 10 mL of PBS) was placed in a water bath (37°C). 342 $\mu$L of 1.0 M L-cysteine (Sigma, St. Louis) in water was added and the reaction mixture let stand at 37°C for 30 minutes. Ammonium sulfate (756 mg) was added and the reaction mixture was loaded onto an HPLC column (Tosohaas, Phenyl 5PW, 10 $\mu$m, 0.75 x 7.5 cm) and eluted with a 60 minute linear gradient (0-100%) of 0.1 M sodium phosphate/0.6 M ammonium sulfate/0.05% EDTA, pH 6.5 to 0.1 M sodium phosphate/0.05% EDTA, pH 6.5 at a flow rate of 6 mL per minute. The peak corresponding to c7E3 Fab' was collected. (PEG$_{2,000}$)$_2$-maleimide (Shearwater Polymers, Inc.) (39.3 mg) was added to the Fab' solution and gently agitated for 2.5 hours at ambient temperature. The reaction mixture was concentrated to 9.8 mL and loaded onto an HPLC column (Tosohaas, Phenyl 5PW, 10 $\mu$m, 0.75 x 7.5 cm) and eluted with a 60 minute linear gradient (0-100%) of 0.1 M sodium phosphate/0.6 M ammonium sulfate, pH 7.5 to 0.1 M sodium phosphate, pH 7.5 at a flow rate of 6 mL per minute. The peak corresponding to the title compound was collected, concentrated and reloaded onto an HPLC column (Tosohaas, Phenyl 5PW, 10 $\mu$m, 0.75 x 7.5 cm) and eluted with a 60 minute linear gradient (50-70%) of 0.1 M sodium phosphate/0.6 M ammonium sulfate, pH 7.5 to 0.1 M sodium phosphate, pH 7.5 at a flow rate of 6 mL per minute. The peak correspond to the titled compound was collected and concentrated to yield 15.6 mg of product (MH$^+$ 57,313.5 (cluster)).

Example XIX Synthesis of c7E3Fab'((PEG$_{5,000}$)$_2$)$_2$

c7E3 F(ab)'$_2$ (54.3 mg in 10mL of PBS) was placed in a water bath (37°C). 342 $\mu$L of 1.0 M L-cysteine (Sigma, St. Louis) in water was added and the reaction mixture let stand at 37°C for 30 minutes. Ammonium sulfate (756 mg) was added and the reaction mixture was loaded onto an HPLC column (Tosohaas, Phenyl 5PW, 10 $\mu$m, 0.75 x 7.5 cm) and eluted with a 60 minute linear gradient (0-100%) of 0.1 M sodium phosphate/0.6 M ammonium sulfate/0.05% EDTA, pH 6.5 to 0.1 M sodium phosphate/0.05% EDTA, pH 6.5 at a flow rate of 6 mL per minute. The peak corresponding to c7E3 Fab' was collected. $(PEG_{5,000})_2$-maleimide (Shearwater Polymers, Inc.) (104 mg) was added to the Fab' solution and gently agitated for 2.5 hours at ambient temperature. The reaction mixture was concentrated to 9.8 mL and loaded onto an HPLC column (Tosohaas, Phenyl 5PW, 10 μm, 0.75 x 7.5 cm) and eluted with a 60 minute linear gradient (0-100%) of 0.1 M sodium phosphate/0.6 M ammonium sulfate, pH 7.5 to 0.1 M sodium phosphate, pH 7.5 at a flow rate of 6 mL per minute. The peak corresponding to the title compound was collected, concentrated and reloaded onto an HPLC column (Tosohaas, Phenyl 5PW, 10 μm, 0.75 x 7.5 cm) and eluted with a 60 minute linear gradient (50-70%) of 0.1 M sodium phosphate/0.6 M ammonium sulfate, pH 7.5 to 0.1 M sodium phosphate, pH 7.5 at a flow rate of 6 mL per minute. The peak correspond to the titled compound was collected and concentrated to yield 14.2 mg of product (MH[+] 71,194(cluster)).

Example XX Synthesis of c7E3Fab'$(PEG_{2,000})_2$

c7E3 F(ab)'$_2$ (40.1 mg in 9.5 mL of PBS) was placed in a water bath (37°C). 163 μL of 1.0 M L-cysteine (Sigma, St. Louis) in water was added and the reaction mixture let stand at 37°C for 30 minutes. Ammonium sulfate (777 mg) was added and the reaction mixture was loaded onto an HPLC column (Tosohaas, Phenyl 5PW, 10 μm, 0.75 x 7.5 cm) and eluted with a 60 minute linear gradient (0-100%) of 0.1 M sodium phosphate/0.6 M ammonium sulfate/0.05% EDTA, pH 6.5 to 0.1 M sodium phosphate/0.05% EDTA, pH 6.5 at a flow rate of 6 mL per minute. The peak corresponding to c7E3 Fab' was collected. $PEG_{2,000}$-maleimide (Shearwater Polymers, Inc.) (20 mg) was added to the Fab' solution and gently agitated for 2.5 hours at ambient temperature. The reaction mixture was concentrated to 9.8 mL and loaded onto an HPLC column (Tosohaas, Phenyl 5PW, 10 μm, 0.75 x 7.5 cm) and eluted with a 60 minute linear gradient (0-100%) of 0.1 M sodium phosphate/0.6 M ammonium sulfate, pH 7.5 to 0.1 M sodium phosphate, pH 7.5 at a flow rate of 6 mL per minute. The peak corresponding to the title compound was collected, concentrated and reloaded onto an HPLC column (Tosohaas, Phenyl 5PW, 10 μm, 0.75 x 7.5 cm) and eluted with a 60 minute linear gradient (50-70%) of 0.1 M sodium phosphate/0.6 M ammonium sulfate, pH 7.5 to 0.1 M sodium phosphate, pH 7.5 at a flow rate of 6 mL per minute. The peak correspond to the titled compound was collected and concentrated to yield 10.4 mg of product (MH[+] 52,817 (cluster)).

Example XXI Inhibition of Platelet Aggregation

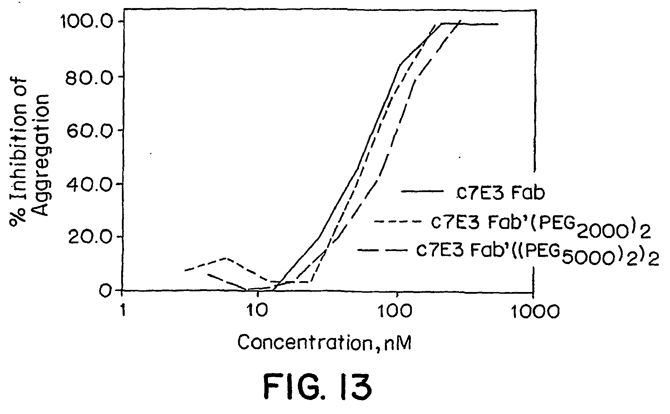

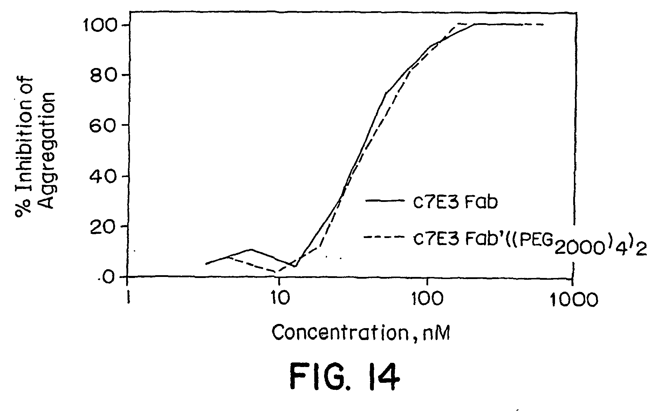

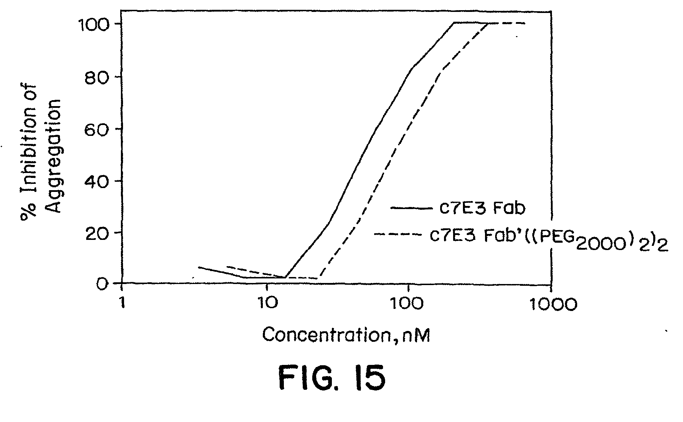

c7E3, c7E3 Fab'$(PEG_{2,000})_2$, c7E3 Fab'$((PEG_{2,000})_2)_2$, c7E3 Fab'$((PEG_{2,000})_4)_2$ and c7E3 Fab'$((PEG_{5,000})_2)_2$ were tested for platelet aggregation inhibiting activity as described in Example IV. The results of these studies are presented graphically in Figures 13-15 and further demonstrate that chemical modification of c7E3 to increase half-life does not affect activity.

Example XXII Determination of the binding kinetics of modified antibodies

Binding kinetics were measured and rate constants and affinities for binding to GPIIb/IIIa were calculated as described in Example XII. The rate constants and affinities reported in Tables 5 (Example XII) and 6 are the average of at least two and up to about seven independent determinations. The data used to calculate the rate constants and affinities reported in Table 5 were included with additional data in calculating the rate constants and affinities reported in Table 6 The rate constants and affinities reported in Table 6 further demonstrate that site specific modification of c7E3 can be accomplished without adversely affect binding, while random modification dramatically reduces affinity.

Table 6 Binding constants

| | $k_a$ (mol$^{-1}$sec$^{-1}$) | $k_d$ (sec$^{-1}$) | $K_D$ (M) |
|---|---|---|---|
| c7E3 Fab | $2.9 \times 10^4$ | $7.6 \times 10^{-5}$ | $3.8 \times 10^{-9}$ |
| c7E3 Fab ($PEG_{5,000}$) | $1.7 \times 10^4$ | $7.7 \times 10^{-5}$ | $4.5 \times 10^{-9}$ |
| c7E3 Fab ($PEG_{20,000}$) | $1.2 \times 10^5$ | $1.9 \times 10^{-4}$ | $1.1 \times 10^{-8}$ |
| c7E3 Fab' $(PEG_{2,000})_2$ | $1.6 \times 10^4$ | $6.8 \times 10^{-5}$ | $2.5 \times 10^{-9}$ |
| c7E3 Fab'$(PEG_{5,000})_2$ | $3.8 \times 10^4$ | $9.8 \times 10^{-5}$ | $3.0 \times 10^{-9}$ |
| c7E3 Fab' $(PEG_{10,000})_2$ | $3.1 \times 10^4$ | $9.6 \times 10^{-5}$ | $3.1 \times 10^{-9}$ |
| c7E3 Fab' $(PEG_{20,000})_2$ | $2.4 \times 10^4$ | $9.0 \times 10^{-5}$ | $4.9 \times 10^{-9}$ |
| c7E3 Fab' $((PEG_{2,000})_2)_2$ | $2.6 \times 10^4$ | $6.8 \times 10^{-5}$ | $2.9 \times 10^{-9}$ |

Table continued

|  | $k_a$ (mol⁻¹sec⁻¹) | $k_d$ (sec⁻¹) | $K_D$ (M) |
|---|---|---|---|
| c7E3 Fab' $((PEG_{5,000})_2)_2$ | $1.4 \times 10^4$ | $7.4 \times 10^{-5}$ | $5.6 \times 10^{-9}$ |
| c7E3 Fab Random $(PEG_{5,000})$ | $8.2 \times 10^3$ | $1.5 \times 10^{-5}$ | $1.9 \times 10^{-8}$ |

Example XXIII Pharmacokinetics in mice

Pharmacokinetics were performed in mice using a protocol similar to those described in Examples IX and X. Mice were given an intravenous bolus of test article, blood was drawn at predetermined time points and the serum concentration of the test article was determined. Seven independent studies using 3-5 mice were conducted. A different test article was administered for each study, as follows: Study 1, c7E3 Fab was administered at a dose of 20 mg/kg; Study 2, c7E3 Fab $PEG_{5000}$ was administered at a dose of 20 mg/kg; Study 3, c7E3 Fab'$((PEG_{2,000})_2)_2$ was administered at a dose of 0.6 mg/kg; Study 4, c7E3 Fab $PEG_{20,000}$ was administered at a dose of 0.65 mg/kg); Study 5, c7E3 Fab'$(PEG_{2,000})_2$ was administered at a dose of 0.56 mg/kg; Study 6, c7E3 Fab'$((PEG_{(5,000)})_2)_2$ was administered at a dose of 0.75 mg/kg; Study 7, c7E3 Fab'$(PEG_{10,000})_2$ was administered at a dose of 0.74 mg/kg. The results of each study are presented in Table 7, and demonstrate that c7E3 Fab $PEG_{5000}$, c7E3 Fab'$((PEG_{2,000})_2)_2$, c7E3 Fab $PEG_{20,000}$, c7E3 Fab'$PEG(C_{5,000})_2)_2$ and c7E3 Fab'$PEG_{(10,000)})_2$ persist in the serum longer than unmodified c7E3 Fab after intravenous administration. These studies also revealed that c7E3 Fab'$(PEG_{2,000})_2$ persists in the serum for about as long as unmodified c7E3 Fab.

Table 7     Pharmacokinetics in mice

| Time (h) | Serum Concentration of Test Article (nM) | | | | | | |
|---|---|---|---|---|---|---|---|
| | Study 1 (c7E3 Fab) | Study 2 (c7E3 Fab PEG$_{5000}$) | Study 3 (c7E3 Fab'((PEG$_{2,000}$)$_2$)$_2$) | Study 4 (c7E3 Fab PEG$_{20,000}$) | Study 5 (c7E3 Fab'(PEG$_{2,000}$)) | Study 6 (c7E3 Fab'((PEG$_{5,000}$)$_2$)$_2$) | Study 7 (c7E3 Fab'(PEG$_{10,000}$)$_2$) |
| 0.17 | 5.7[1] | 6.0 | nd | nd | nd | nd | nd |
| 1 | 1.3 | 5.9 | 91.3 | 92.4 | 63.3 | 228.7 | 303.9 |
| 6 | nd | 1.5 | 43.1 | 73.3 | 3.0 | 160.3 | 130.2 |
| 24 | nd | nd | 2.9 | 17.7 | 0.2 | 44.8 | 79.4 |
| 48 | nd | nd | 1.0 | 10.4 | 0.0 | 34.9 | 29.6 |
| 72 | nd | nd | 0.2 | 3.0 | nd | 11.0 | 15.6 |
| 96 | nd | nd | 0.1 | nd | nd | 5.2 | 13.0 |

nd, not determined

Example XXIV Pharmacodynamics in cynomologus monkeys

Seven independent studies using 2-4 cynomologus monkeys were conducted. Each animal was instrumented with a vascular access port (VAPs) for obtaining blood samples. An initial blood sample was acquired and platelet aggregation was measured at the time of VAP placement. Each animal was administered a single bolus of test article by intravenous injection. A different test article was administered for each study, as follows: Study 1, c7E3 Fab was administered at a dose of 0.4 mg/kg; Study 2, c7E3 Fab'(PEG$_{5,000}$)$_2$ was administered at a dose of 1.05 mg/kg; Study 3, c7E3 Fab'(PEG$_{10,000}$)$_2$ was administered at a dose of 0.6 mg/kg; Study 4, c7E3 Fab'(PEG$_{5,000}$)$_2$)$_2$ was administered at a dose of 0.6 mg/kg; Study 5, c7E3 Fab'(PEG$_{20,000}$)$_2$ was administered at a dose of 1.4 mg/kg; Study 6, c7E3 Fab'(PEG$_{2,000}$)$_2$ was administered at a dose of 0.45 mg/kg. Blood was collected prior to administration of test article and at predetermined time points, for assessment of platelet function, hematology and pharmacokinetics. Platelet aggregation was measured as described in Example IV. Serum levels of test article were evaluated as described in Example VIII. The results of the studies are presented in Tables 8a and 8b.

The studies revealed that platelet aggregation was inhibited by 96% one hour after administration of c7E3 Fab, but that inhibition declined rapidly thereafter. The amount of c7E3 which was detected in the serum also rapidly declined after one hour, paralleling the inhibition of platelet aggregation. Similar results were obtained when c7E3 Fab'(PEG$_{2,000}$)$_2$ was administered, demonstrating that modification with two linear PEG chains of molecular weight 2,000 did not increase serum persistence in comparison with the unmodified Fab. In contrast, the administration of Fab' fragments which were modified with PEG chains of molecular weight greater than 2,000 resulted in prolonged inhibition of platelet aggregation and a slower decline in inhibitory activity. Similarly, the quantity of Fab' fragments modified with PEG chains of molecular weight greater than 2,000 which were detected in serum one hour after administration was higher than for the unmodified Fab', and the serum levels declined at a slower rate.

### Table 8a        Phamacodynamics in monkeys

| | Study 1 (c7E3 Fab) | | Study 2 (c7E3 Fab'(PEG$_{5,000}$)$_2$) | | Study 3 (c7E3 Fab'(PEG$_{10,000}$)$_2$) | |
|---|---|---|---|---|---|---|
| Time (hours) | platelet aggregation (%inhibition) | serum concentration (nM) | platelet aggregation (% inhibition) | serum concentration (nM) | platelet aggregation (% inhibition) | serum concentration (nM) |
| 0.17 | nd | nd | nd | nd | 88 | 58.9 |
| 1 | 96 | 28.8 | 100 | 285.0 | 86 | 43.1 |
| 6 | 29 | 1.9 | 99 | 145.6 | nd | nd |
| 12 | 15 | 1.0 | 77 | 88.3 | 60 | 15.0 |
| 24 | 5 | 0.5 | 32 | 29.4 | 28 | 8.1 |
| 36 | 7 | 0.3 | 23 | 15.6 | 28 | 5.2 |
| 48 | 1 | 0.2 | 22 | 10.5 | 26 | 3.0 |

nd, not determined

EP 1 144 452 B1

Table 8b        Pharmacodynamics in monkeys

| Time (hours) | Study 4 (c7E3 Fab'((PEG$_{5,000}$)$_2$)$_2$) | | Study 5 (c7E3 Fab'(PEG$_{20,000}$)$_2$) | | Study 6 (c7E3 Fab'(PEG$_{2,000}$)$_2$) | |
|---|---|---|---|---|---|---|
| | platelet aggregation (%inhibition) | serum concentration (nM) | platelet aggregation (% inhibition) | serum concentration (nM) | platelet aggregation (% inhibition) | serum concentration (nM) |
| 1 | 73 | 89.9 | 100 | 185.4 | 75 | 59.7 |
| 4 | nd | nd | 100 | 128.2 | nd | nd |
| 6 | 59 | 50.5 | nd | nd | 36 | 10.5 |
| 8 | nd | nd | 100 | 96.7 | nd | nd |
| 12 | 30 | 30.0 | 100 | 80.4 | 19 | 3.5 |
| 24 | 11 | 19.7 | 99 | 49.6 | 15 | 1.8 |
| 36 | 0 | 11.5 | nd | nd | 12 | 1.1 |
| 48 | 0 | 7.5 | 77 | 21.7 | 17 | 0.8 |
| 120 | nd | nd | 7 | 3.9 | nd | nd |

nd, not determined

Example XXV c7E3 Fab-PEG$_{20,000}$

The titled compound was prepared as described in Example III except substituting PEG$_{20,000}$ aldehyde for PEG$_{5,000}$ aldehyde.

## Claims

1. A modified antibody or modified antigen-binding fragment that specifically binds to one or more antigens selected from the group consisting of GPIIb/IIIa, $\alpha_v\beta_3$ and Mac-1, wherein said modified antibody or antigen-binding fragment comprises one to six organic moieties, which are covalently bonded to said antibody or antigen-binding fragment, and which are each, independently, a substituted hydrophilic polymeric group that is substituted with a lipid or phospholipid.

2. The modified antibody or modified antigen-binding fragment of claim 1 wherein an organic moiety is covalently bonded to at least one carboxyl-terminus of said antibody or antigen-binding fragment.

3. The modified antibody or modified antigen-binding fragment of any one of the preceding claims wherein said modified antibody or modified antigen-binding fragment is a modified antigen-binding fragment selected from the group consisting of a modified Fab' fragment and a modified Fab fragment.

4. The modified antibody or modified antigen-binding fragment of claim 3 wherein said modified antibody or modified antigen-binding fragment is a modified antigen-binding fragment that is an achromopeptidase fragment.

5. The modified antibody or modified antigen-binding fragment of any one of the preceding claims wherein an organic moiety is bonded to the carboxyl-terminus of the heavy chain.

6. The modified antibody or modified antigen-binding fragment of any one of the preceding claims wherein an organic moiety is covalently bonded to the side chain sulfur atom of a cysteinyl residue of the antibody or antigen-binding fragment.

7. The modified antibody or modified antigen-binding fragment of claim 6 wherein the heavy chain comprises said cysteinyl residue.

8. The modified antibody or modified antigen-binding fragment of claim 7 wherein said modified antibody or modified antigen-binding fragment is an Fab' fragment and said organic moiety is bonded to a sulfur atom of a heavy chain cysteinyl residue that forms an inter-chain disulfide bond.

9. The modified antibody or modified antigen-binding fragment of any one of the preceding claims that specifically binds GPIIb/IIIa;
inhibits binding of fibrinogen to GPIIb/IIIa;
and optionally further specifically binds $\alpha_v\beta_3$.

10. The modified antibody or modified antigen-binding fragment of Claim 9 wherein the binding of said antibody or antigen-binding fragment to GPIIb/IIIa and/or $\alpha_v\beta_3$ is competitively inhibited by the 7E3 antibody produced by the hybridoma cell line deposited as ATCC Accession Number HB 8832.

11. The modified antibody or modified antigen-binding fragment of any one of the preceding claims wherein said antibody or antigen-binding fragment is **characterized by** increased in vivo serum half-life, as compared to the corresponding unmodified antibody or antigen-binding fragment.

12. The modified antibody or modified antigen-binding fragment of any one of the preceding claims wherein said antibody or antigen-binding fragment binds GPIIb/IIIa and/or $\alpha_v\beta_3$ with substantially the same affinity as the corresponding unmodified antibody or antigen-binding fragment.

13. The modified antibody or modified antigen-binding fragment of any one of the preceding claims wherein said antibody or antigen-binding fragment is **characterized by** decreased immunogenicity, as compared to the corresponding unmodified antibody or antigen-binding fragment.

14. The modified antibody or modified antigen-binding fragment of any one of the preceding claims wherein said antibody or antigen-binding fragment is selected from the group consisting of the 7E3 antibody, a humanized 7E3 antibody, a chimeric 7E3 antibody, and an antigen-binding fragment of any of the foregoing, wherein said 7E3 antibody is the antibody produced by the hybridoma cell line deposited as ATCC Accession Number HB 8832.

15. The modified antibody or modified antigen-binding fragment of any one of the preceding claims wherein said substituted hydrophilic polymeric group is a substituted linear or branched polyalkane glycol chain with a molecular weight greater than 2,000 Daltons.

16. The modified antibody or modified antigen-binding fragment of any one of the preceding claims wherein said substituted hydrophilic polymeric group is a linear or branched substituted polyethylene glycol chain.

17. The modified antibody or modified antigen-binding fragment of any one of the preceding claims wherein said antibody or antigen-binding fragment is c7E3 or an antigen-binding fragment thereof.

18. The modified antibody or modified antigen-binding fragment of any one of the preceding claims wherein said substituted hydrophilic polymeric group is a linear or branched substituted polyethylene glycol chain that is substituted or terminally substituted with a phospholipid group.

19. The modified antibody or modified antigen-binding fragment of claim 18 wherein said phospholipid group has the structural formula:

$$-NH-CH_2-CH_2-O-P(O^-)(O)-O-CH_2-CH(R)-CH_2-R';$$

wherein R and R' are each, independently, -OH or a group represented by -X-Y, wherein - X- is -O-, -O-C(O)-, -S-, -O-S(O)$_2$-O-, -NH-C(O)- or -O-CH=CH-, and Y is a saturated or unsaturated $C_6$ to $C_{40}$ hydrocarbon group, and wherein both R and R' are not -OH.

20. The modified antibody or modified antigen-binding fragment of claim 19, wherein:

said antibody or antigen-binding fragment is c7E3 or an antigen-binding fragment of c7E3; and
the substituted hydrophilic polymeric group is a linear or branched 3,400 Dalton polyethylene glycol chain that is terminally substituted with a phospholipid.

21. The modified antibody or modified antigen-binding fragment of any one of claims 18-20 wherein said antibody or antigen-binding fragment is an antigen-binding fragment selected from the group consisting of an Fab' fragment and an Fab fragment.

22. The modified antibody or modified antigen-binding fragment of claim 21 wherein said antibody or antigen-binding fragment is an Fab' fragment.

23. The modified antibody or modified antigen-binding fragment of claim 14 wherein said antibody or antigen-binding fragment is a humanized 7E3 antibody or antigen-binding fragment thereof comprising:

   a human constant region or portion thereof;
   CDR1, CDR2 and CDR3 of the light chain of the 7E3 antibody; and
   CDR1, CDR2 and CDR3 of the heavy chain of the 7E3 antibody,

wherein the 7E3 antibody is the antibody produced by the hybridoma cell line deposited as ATCC Accession Number HB 8832.

24. The modified antibody or modified antigen-binding fragment of claim 14 wherein said antibody or antigen-binding fragment is a humanized 7E3 antibody or antigen-binding fragment thereof.

25. The modified antibody or modified antigen-binding fragment of claim 14 wherein said antibody or antigen-binding fragment is a chimeric 7E3 antibody or antigen-binding fragment thereof.

26. Use of a modified antibody or modified antigen-binding fragment according to any one of the preceding claims for the manufacture of a medicament for the treatment of thrombosis in a patient, or inhibition of stenosis and/or restenosis following a vascular intervention procedure, or prevention or reduction of ischemia, or inhibition of the growth and/or metastasis of a tumor.

27. Use of claim 26 wherein said modified antibody or modified antigen-binding fragment inhibits platelet mediated metastasis.

28. Use of a modified antibody or modified antigen-binding fragment according to any one of the preceding claims for the manufacture of a medicament for inhibiting $\alpha_v\beta_3$-mediated angiogenesis in a human, wherein said modified antibody or modified antigen-binding fragment specifically binds $\alpha_v\beta_3$.

29. An *in vitro* method of inhibiting a process mediated by the binding of a ligand to GPIIb/IIIa, $\alpha_v\beta_3$ and/or Mac-1 expressed on the plasma membrane of a cell, comprising contacting the cell with an effective amount of a modified antibody or antigen-binding fragment of any one of claims 1 to 25.


**Patentansprüche**

1. Modifizierter Antikörper oder modifiziertes, Antigen-bindendes Fragment, das spezifisch an ein oder mehrere Antigene bindet ausgewählt aus der Gruppe bestehend aus GPIIb/IIIa, $\alpha_v\beta_3$ und Mac-1, worin der modifizierte Antikörper oder das Antigen-bindende Fragment ein bis sechs organische Reste umfasst, die an den Antikörper oder das Antigen-bindende Fragment kovalent gebunden sind und die jeweils unabhängig eine substituierte, hydrophile, polymere Gruppe sind, welche mit einem Lipid oder einem Phospholipid substituiert ist.

2. Modifizierter Antikörper oder modifiziertes Antigen-bindendes Fragment nach Anspruch 1, worin ein organischer Rest an mindestens einem Carboxyl-Terminus des Antikörpers oder des Antigen-bindenden Fragments kovalent gebunden ist.

3. Modifizierter Antikörper oder modifiziertes Antigen-bindendes Fragment nach einem der vorstehenden Ansprüche, worin der modifizierte Antikörper oder das modifizierte Antigen-bindende Fragment ein modifiziertes Antigen-bindendes Fragment ist ausgewählt aus der Gruppe bestehend aus einem modifizierten Fab'-Fragment und einem modifizierten Fab-Fragment.

4. Modifizierter Antikörper oder modifiziertes Antigen-bindendes Fragment nach Anspruch 3, worin der modifizierte Antikörper oder das modifizierte Antigen-bindende Fragment ein modifiziertes, Antigen-bindendes Fragment ist, das ein Achromopeptidase-Fragment ist.

5. Modifizierter Antikörper oder modifiziertes Antigen-bindendes Fragment nach einem der vorstehenden Ansprüche, worin ein organischer Rest an den Carboxyl-Terminus der schweren Kette gebunden ist.

6. Modifizierter Antikörper oder modifiziertes Antigen-bindendes Fragment nach einem der vorstehenden Ansprüche, worin ein organischer Rest kovalent an das Seitenketten-Schwefelatom eines Cysteinyl-Restes des Antikörpers oder des Antigen-bindenden Fragments gebunden ist.

7. Modifizierter Antikörper oder modifiziertes Antigen-bindendes Fragment nach Anspruch 6, worin die schwere Kette den Cysteinyl-Rest umfasst.

8. Modifizierter Antikörper oder modifiziertes Antigen-bindendes Fragment nach Anspruch 7, worin der modifizierte Antikörper oder das modifizierte Antigen-bindende Fragment ein Fab'-Fragment ist und worin der organische Rest an ein Schwefelatom eines Cysteinyl-Restes einer schweren Kette gebunden ist, der eine Inter-Ketten-Disulfidbrücke bildet.

9. Modifizierter Antikörper oder modifiziertes Antigen-bindendes Fragment nach einem der vorstehenden Ansprüche welcher/welches

spezifisch an GPIIb/IIIa bindet;
Bindung von Fibrinogen an GPII/IIIa inhibiert;
und wahlweise weiter spezifisch $\alpha_v\beta_3$ bindet.

10. Modifizierter Antikörper oder modifiziertes Antigen-bindendes Fragment nach Anspruch 9, worin die Bindung des Antikörpers oder des Antigen-bindenden Fragments an GPIIb/IIIa und/oder $\alpha_v\beta_3$ durch den 7E3 Antikörper kompetitiv inhibiert wird, welcher von der mit der ATCC Hinterlegungsnummer HB 8832 hinterlegten Hybridomzelllinie hergestellt wird.

11. Modifizierter Antikörper oder modifiziertes Antigen-bindendes Fragment nach einem der vorstehenden Ansprüche, worin der Antikörper oder das Antigen-bindende Fragment **gekennzeichnet ist durch** eine verglichen mit dem entsprechenden nicht-modifizierten Antikörper oder dem nicht-modifizierten Antigen-bindenden Fragment erhöhte in vivo Serum-Halbwertzeit.

12. Modifizierter Antikörper oder modifiziertes Antigen-bindendes Fragment nach einem der vorstehenden Ansprüche, worin der Antikörper oder das Antigen-bindende Fragment GPIIb/IIIa und/oder $\alpha_v\beta_3$ mit im wesentlichen der gleichen Affinität bindet, wie der entsprechende nicht-modifizierte Antikörper oder das nicht-modifizierte Antigen-bindende Fragment.

13. Modifizierter Antikörper oder modifiziertes Antigen-bindendes Fragment nach einem der vorstehenden Ansprüche, worin der Antikörper oder das Antigen-bindende Fragment **gekennzeichnet ist durch** eine verglichen mit dem entsprechenden nicht-modifizierten Antikörper oder dem nicht-modifizierten Antigen-bindenden Fragment verringerten Immunogenizität.

14. Modifizierter Antikörper oder modifiziertes Antigen-bindende Fragment nach einem der vorstehenden Ansprüche, worin der Antikörper oder das Antigen-bindende Fragment ausgewählt ist aus der Gruppe bestehend aus dem 7E3 Antikörper, einem humanisierten 7E3 Antikörper, einem chimären 7E3 Antikörper und einem Antigen-bindenden Fragment von einem der Vorstehenden, worin der 7E3 Antikörper der Antikörper ist, der von der mit der ATCC Hinterlegungsnummer HB 8832 hinterlegten Hybridomzelllinie produziert wird.

15. Modifizierter Antikörper oder modifiziertes Antigen-bindendes Fragment nach einem der vorstehenden Ansprüche, worin die substituierte hydrophile polymere Gruppe eine substituierte lineare oder verzweigte Polyalkanglykol-Kette mit einem Molekulargewicht von über 2000 Dalton ist.

**16.** Modifizierter Antikörper oder modifiziertes Antigen-bindendes Fragment nach einem der vorstehenden Ansprüche, worin die substituierte hydrophile polymere Gruppe eine lineare oder verzweigte substituierte Polyethylenglykol Kette ist.

**17.** Modifizierter Antikörper oder modifiziertes Antigen-bindendes Fragment nach einem der vorstehenden Ansprüche, worin der Antikörper oder das Antigen-bindende Fragment c7E3 oder ein Antigen-bindendes Fragment davon ist.

**18.** Modifizierter Antikörper oder modifiziertes Antigen-bindendes Fragment nach einem der vorstehenden Ansprüche, worin die substituierte hydrophile polymere Gruppe eine lineare oder verzweigte substituierte Polyethylenglykol-Kette ist, die substituiert oder mit einer Phospholipid-Gruppe terminal substituiert ist.

**19.** Modifizierter Antikörper oder modifiziertes Antigen-bindendes Fragment nach Anspruch 18, worin die Phospholipidgruppe die folgende Strukturform aufweist:

$$-NH-CH_2-CH_2-O-P(O^-)(O)-O-CH_2-CH(R)-CH_2-R';$$

worin R und R' jeweils unabhängig sind, -OH oder eine durch -X-Y dargestellte Gruppe, worin -X- ist, -O-, -O-C(O)-, -S-, -O-S(O)$_2$-O-, -NH-C(O)- oder -O-CH=CH-, und worin Y eine gesättigte oder ungesättigte $C_6$-$C_{40}$-Kohlenwasserstoffgruppe ist, und worin R und R' nicht beide -OH sind.

**20.** Modifizierter Antikörper oder modifiziertes Antigen-bindendes Fragment nach Anspruch 19, worin der Antikörper oder das Antigen-bindende Fragment c7E3 oder ein Antigen-bindendes Fragment von c7E3 ist; und worin die substituierte hydrophile polymere Gruppe eine lineare oder verzweigte Polyethylenglycol-Kette mit 3400 Dalton ist, welche mit einem Phospholipid terminal substituiert ist.

**21.** Modifizierter Antikörper oder modifiziertes Antigen-bindendes Fragment nach einem der Ansprüche 18-20, worin der Antikörper oder das Antigen-bindende Fragment ein Antigen-bindendes Fragment ist ausgewählt aus der Gruppe bestehend aus einem Fab'-Fragment und einem Fab-Fragment.

**22.** Modifizierter Antikörper oder modifiziertes Antigen-bindendes Fragment nach Anspruch 21, worin der Antikörper oder das Antigen-bindende Fragment ein Fab'-Fragment ist.

**23.** Modifizierter Antikörper oder modifiziertes Antigen-bindende Fragment nach Anspruch 14, worin der Antikörper oder das Antigen-bindende Fragment ein humanisierter 7E3 Antikörper oder ein Antigen-bindendes Fragment davon ist, umfassend:

eine menschliche konstante Region oder ein Teil davon;
CDR1, CDR2 und CDR3 der leichten Kette des 7E3 Antikörper; und
CDR1, CDR2 und CDR3 der schweren Kette des 7E3 Antikörpers,
worin der 7E3 Antikörper der von der mit der ATCC Hinterlegungsnummer HB 8832 hinterlegten Hybridomzelllinie produzierte Antikörper ist.

**24.** Modifizierter Antikörper oder modifiziertes Antigen-bindendes Fragment nach Anspruch 14, worin der Antikörper oder das Antigen-bindende Fragment ein humanisierter 7E3 Antikörper oder Antigen-bindendes Fragment davon ist.

**25.** Modifizierter Antikörper oder modifiziertes Antigen-bindendes Fragment nach Anspruch 14, worin der Antikörper oder das Antigen-bindende Fragment ein chimärer 7E3 Antikörper oder Antigen-bindendes Fragment davon ist.

**26.** Verwendung eines modifizierten Antikörpers oder modifizierten Antigen-bindenden Fragments nach einem der vorstehenden Ansprüche zur Herstellung eines Arzneimittels zur Behandlung von Thrombose in einem Patienten, oder zur Inhibierung von Stenose und/oder Restenose nach einer Gefäßbehandlung, oder zur Verhinderung oder Reduzierung von Ischämie, oder zur Inhibierung des Wachstums und/oder Metastasierung eines Tumors.

**27.** Verwendung Anspruch 26, wobei der modifizierte Antikörper oder das modifizierte Antigen-bindende Fragment Platelet vermittelte Metastase inhibiert.

**28.** Verwendung eines modifizierten Antikörpers oder modifizierten Antigen-bindenden Fragments nach einem der vorstehenden Ansprüche zur Herstellung eines Arzneimittels zur Inhibierung von $\alpha_v\beta_3$ vermittelter Angiogenese in

einem Menschen, worin der modifizierte Antikörper oder das modifizierte Antigen-bindende Fragment spezifisch an $\alpha_v\beta_3$ bindet.

29. In vitro Verfahren zur Inhibierung eines durch die Bindung eines Liganden an GPIIb/IIIa, $\alpha_v\beta_3$ und/oder MAC-1, exprimiert auf der Plasma-Membran einer Zelle, vermittelten Ablaufs, umfassend, Inkontaktbringen der Zelle mit einer wirksamen Menge eines modifizierten Antikörpers oder Antigen-bindenden Fragments nach einem der Ansprüche 1 bis 25.

**Revendications**

1. Anticorps modifié ou fragment modifié de liaison à un antigène qui se lie de manière spécifique à un ou plusieurs antigènes choisis parmi le groupe constitué par GPIIb/IIIa, $\alpha_v\beta_3$ et Mac-1, dans lequel ledit anticorps modifié ou ledit fragment modifié de liaison à un antigène comprend de une à six fractions organiques qui sont liées de manière covalente audit anticorps ou audit fragment de liaison à un antigène et qui représentent, chacun indépendamment l'un de l'autre, un groupe polymère hydrophile substitué qui est substitué avec un lipide ou avec un phospholipide.

2. Anticorps modifié ou fragment modifié de liaison à un antigène selon la revendication 1, dans lequel une fraction organique est liée de manière covalente à au moins une terminaison carboxyle dudit anticorps ou dudit fragment de liaison à un antigène.

3. Anticorps modifié ou fragment modifié de liaison à un antigène selon l'une quelconque des revendications précédentes, dans lequel ledit anticorps modifié ou ledit fragment modifié de liaison à un antigène est un fragment modifié de liaison à un antigène choisi parmi le groupe constitué par un fragment Fab' modifié et un fragment Fab modifié.

4. Anticorps modifié ou fragment modifié de liaison à un antigène selon la revendication 3, dans lequel ledit anticorps modifié ou ledit fragment modifié de liaison à un antigène est un fragment modifié de liaison à un antigène, à savoir un fragment d'achromopeptidase.

5. Anticorps modifié ou fragment modifié de liaison à un antigène selon l'une quelconque des revendications précédentes, dans lequel une fraction organique est liée à la terminaison carboxyle de la chaîne lourde.

6. Anticorps modifié ou fragment modifié de liaison à un antigène selon l'une quelconque des revendications précédentes, dans lequel une fraction organique est liée de manière covalente à l'atome de soufre de la chaîne latérale d'un résidu cystéinyle de l'anticorps ou du fragment de liaison à un antigène.

7. Anticorps modifié ou fragment modifié de liaison à un antigène selon la revendication 6, dans lequel la chaîne lourde comprend ledit résidu cystéinyle.

8. Anticorps modifié ou fragment modifié de liaison à un antigène selon la revendication 7, dans lequel ledit anticorps modifié ou ledit fragment modifié de liaison à un antigène est un fragment Fab' et ladite fraction organique est liée à un atome de soufre d'un résidu cystéinyle de chaîne lourde qui forme une liaison disulfure intercaténaire.

9. Anticorps modifié ou fragment modifié de liaison à un antigène selon l'une quelconque des revendications précédentes, qui
se lie de manière spécifique à GPIIb/IIIa ;
inhibe la liaison du fibrinogène à GPIIb/IIIa ;
et le cas échéant, se lie en outre de manière spécifique à $\alpha_v\beta_3$.

10. Anticorps modifié ou fragment modifié de liaison à un antigène selon la revendication 9, dans lequel la liaison dudit anticorps ou dudit fragment de liaison à un antigène à GPIIb/IIIa et/ou à $\alpha_v\beta_3$ est soumise à une inhibition compétitive via l'anticorps 7E3 produit par la lignée cellulaire d'hybridomes déposée sous le numéro matricule ATCC HB 8832.

11. Anticorps modifié ou fragment modifié de liaison à un antigène selon l'une quelconque des revendications précédentes, dans lequel ledit anticorps ou ledit fragment de liaison à un antigène est **caractérisé par** une augmentation de sa demi-vie sérique in vivo, par rapport à celle de l'anticorps ou du fragment de liaison à un antigène correspondant qui n'a pas été modifié.

**12.** Anticorps modifié ou fragment modifié de liaison à un antigène selon l'une quelconque des revendications précédentes, dans lequel ledit anticorps ou ledit fragment de liaison à un antigène se lie à GPIIb/IIIa et/ou à $\alpha_v\beta_3$ avec essentiellement la même affinité que celle de l'anticorps ou du fragment de liaison à un antigène correspondant qui n'a pas été modifié.

**13.** Anticorps modifié ou fragment modifié de liaison à un antigène selon l'une quelconque des revendications précédentes, dans lequel ledit anticorps ou ledit fragment de liaison à un antigène est **caractérisé par** une diminution de l'immunogénicité, par comparaison avec l'anticorps ou avec le fragment de liaison à un antigène correspondant qui n'a pas été modifié.

**14.** Anticorps modifié ou fragment modifié de liaison à un antigène selon l'une quelconque des revendications précédentes, dans lequel ledit anticorps ou ledit fragment de liaison à un antigène est choisi parmi le groupe constitué par l'anticorps 7E3, un anticorps 7E3 humanisé, un anticorps 7E3 chimère, et un fragment de liaison à un antigène de l'un quelconque des anticorps indiqués ci-dessus, ledit anticorps 7E3 étant un anticorps produit par la lignée cellulaire d'hybridomes déposée sous le numéro matricule ATCC HB 8832.

**15.** Anticorps modifié ou fragment modifié de liaison à un antigène selon l'une quelconque des revendications précédentes, dans lequel ledit groupe polymère hydrophile substitué est une chaîne linéaire ou ramifiée substituée de polyalcaneglycol dont le poids moléculaire est supérieur à 2000 Daltons.

**16.** Anticorps modifié ou fragment modifié de liaison à un antigène selon l'une quelconque des revendications précédentes, dans lequel ledit groupe polymère hydrophile substitué est une chaîne linéaire ou ramifiée d'un polyéthylèneglycol substitué.

**17.** Anticorps modifié ou fragment modifié de liaison à un antigène selon l'une quelconque des revendications précédentes, dans lequel ledit anticorps ou ledit fragment de liaison à un antigène est c7E3 ou un de ses fragments de liaison à un antigène.

**18.** Anticorps modifié ou fragment modifié de liaison à un antigène selon l'une quelconque des revendications précédentes, dans lequel ledit groupe polymère hydrophile substitué est une chaîne linéaire ou ramifiée d'un polyéthylèneglycol substitué qui est substituée ou substituée en position terminale par un groupe phospholipide.

**19.** Anticorps modifié ou fragment modifié de liaison à un antigène selon la revendication 18, dans lequel ledit groupe phospholipide répond à la formule développée :

$$-NH-CH_2-CH_2-O-P(O^-)(O)-O-CH_2-CH(R)-CH_2-R';$$

dans laquelle R et R' représentent, chacun indépendamment l'un de l'autre, un groupe -OH ou un groupe représenté par -X-Y où -X- représente un groupe -O-, un groupe -O-C(O)-, un groupe -S-, un groupe -O-S(O)$_2$-O-, un groupe -NH-C(O)- ou un groupe -O-CH=CH-, et Y représente un groupe d'hydrocarbure saturé ou insaturé contenant de 6 à 40 atomes de carbone, et dans laquelle les deux radicaux R et R' ne représentent pas un groupe -OH.

**20.** Anticorps modifié ou fragment modifié de liaison à un antigène selon la revendication 19, dans lequel ledit anticorps ou ledit fragment de liaison à un antigène est c7E3 ou un fragment de c7E3 de liaison à un antigène ; et le groupe polymère hydrophile substitué est une chaîne linéaire ou ramifiée de polyéthylèneglycol de 3400 Daltons soumise à une substitution en position terminale avec un phospholipide.

**21.** Anticorps modifié ou fragment modifié de liaison à un antigène selon l'une quelconque des revendications 18 à 20, dans lequel ledit anticorps ou ledit fragment de liaison à un antigène est un fragment de liaison à un antigène choisi parmi le groupe constitué par un fragment Fab' ou un fragment Fab.

**22.** Anticorps modifié ou fragment modifié de liaison à un antigène selon la revendication 21, dans lequel ledit anticorps ou ledit fragment de liaison à un antigène est un fragment Fab'.

**23.** Anticorps modifié ou fragment modifié de liaison à un antigène selon la revendication 14, dans lequel ledit anticorps ou ledit fragment de liaison à un antigène est un anticorps 7E3 humanisé ou un de ses fragments de liaison à un antigène, comprenant :

une région humaine constante ou une portion de ladite région ;
CDR1, CDR2 et CDR3 de la chaîne légère de l'anticorps 7E3 ; et
CDR1, CDR2 et CDR3 de la chaîne lourde de l'anticorps 7E3,

dans lequel l'anticorps 7E3 est l'anticorps produit par la lignée cellulaire d'hybridomes déposée sous le numéro matricule ATCC HB 8832.

**24.** Anticorps modifié ou fragment modifié de liaison à un antigène selon la revendication 14, dans lequel ledit anticorps ou ledit fragment de liaison à un antigène est un anticorps 7E3 humanisé ou un de ses fragments de liaison à un antigène.

**25.** Anticorps modifié ou fragment modifié de liaison à un antigène selon la revendication 14, dans lequel ledit anticorps ou ledit fragment de liaison à un antigène est un anticorps 7E3 chimère ou un de ses fragments de liaison à un antigène.

**26.** Utilisation d'un anticorps modifié ou d'un fragment modifié de liaison à un antigène selon l'une quelconque des revendications précédentes pour la préparation d'un médicament destiné au traitement d'une thrombose chez un patient, ou à l'inhibition d'une sténose et/ou d'une resténose suite à une opération chirurgicale de type vasculaire, ou à la prévention ou à la réduction d'une ischémie, ou à l'inhibition de la croissance et/ou de la métastase d'une tumeur.

**27.** Utilisation selon la revendication 26, dans laquelle ledit anticorps modifié ou ledit fragment modifié de liaison à un antigène inhibe la métastase à médiation plaquettaire.

**28.** Utilisation d'un anticorps modifié ou d'un fragment modifié de liaison à un antigène selon l'une quelconque des revendications précédentes pour la préparation d'un médicament destiné à inhiber l'angiogenèse à médiation $\alpha_v\beta_3$ chez l'homme, ledit anticorps modifié ou ledit fragment modifié de liaison à un antigène se liant de manière spécifique à $\alpha_v\beta_3$.

**29.** Procédé in vitro d'inhibition d'un processus dont le médiateur est la liaison d'un ligand à GPIIb/IIIa, $\alpha_v\beta_3$ et/ou Mac-1 exprimés sur la membrane plasmatique d'une cellule, comprenant la mise en contact de la cellule avec une quantité efficace d'un anticorps modifié ou d'un fragment modifié de liaison à un antigène selon l'une quelconque des revendications 1 à 25.

Figure 1

Figure 2

Figure 3

Figure 4

Figure 5

Figure 6.

Figure 7

EP 1 144 452 B1

Figure 8

Figure 9

EP 1 144 452 B1

Figure 10

Figure 11

Figure 12

FIG. 13

FIG. 14

FIG. 15